# EUROPÄISCHE PATENTSCHRIFT

(11) **EP 0 571 414 B1**
(45) Veröffentlichungstag und Bekanntmachung des Hinweises auf die Patenterteilung: **18.09.1996**
(21) Anmeldenummer: 92903641.6
(22) Anmeldetag: 01.02.1992
(51) Int. Cl.: A61K 47/48

(54) **NEUE, ÜBER ENDOZYTOSE IN HÖHERE EUKARYOTISCHE ZELLEN AUFNEHMBARE, NUKLEINSÄURE ENTHALTENDE KOMPLEXE**
NOVEL COMPLEXES CONTAINING NUCLEIC ACID WHICH CAN BE ABSORBED INTO HIGHER EUCARYOTIC CELLS VIA ENDOCYTOSIS
NOUVEAUX COMPLEXES CONTENANT DE L'ACIDE NUCLEIQUE ABSORBABLES PAR ENDOCYTOSE PAR DES CELLULES EUCARYOTES SUPERIEURES

(30) Priorität: 12.02.1991 DE 4104186
(43) Veröffentlichungstag der Anmeldung: 01.12.1993
(73) Patentinhaber: BOEHRINGER INGELHEIM INTERNATIONAL GmbH, D-55216 Ingelheim am Rhein (DE); GENENTECH, INC., South San Francisco California 94080 (US)
(72) Erfinder: BIRNSTIEL, Max, L., A-1100 Wien (AT); COTTEN, Matthew, A-1130 Wien (AT); WAGNER, Ernst, A-2103 Langenzersdorf (AT)
(74) Vertreter: Laudien, Dieter, Dr.
(86) Internationale Anmeldenummer: EP9200217
(87) Internationale Veröffentlichungsnummer: WO9213570

(56) Entgegenhaltungen:
- EP-A- 0 387 775
- EP-A- 0 388 758
- WO-A-91/17773
- Proc. Natl. Acad. Sci. USA, Band 87, Mai 1990, Washington, DC, US; E. WAGNER et al.: "Transferrin-polycation conjugates as carriers for DNA uptake into cells", Seiten 3410-3414
- Bioconjugate Chem., Band 2, Nr. 4, Juli/August 1991, American Chemical Society, Washington, DC, US; E. WAGNER et al.: "DNA-binding transferrin conjugates as functional gene-delivery agents: synthesis by linkage of polylysine or ethidium homodimer to the transferrin carbohydrate moiety", Seiten 226-231

## Beschreibung

Die Erfindung bezieht sich auf das Einbringen von Nukleinsäuren über Endozytose in höhere eukaroytische Zellen mittels Internalisierungsfaktoren.

Nukleinsäuren als therapeutisch wirksame Substanzen haben in den letzten Jahren zunehmend an Bedeutung gewonnen, z.B. haben sich Antisense RNAs und -DNAs als wirksame Mittel für die selektive Inhibierung bestimmter Gensequenzen erwiesen. Ihre Wirkungsweise ermöglicht ihre Anwendung als Therapeutika zur Blockierung der Expression bestimmter Gene (wie deregulierter Onkogene oder viraler Gene) in vivo. Es wurde bereits gezeigt, daß kurze Antisense-Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Ein weiterer Ansatz zur selektiven Inhibierung von Genen besteht in der Anwendung von Ribozymen. Auch hier besteht das Bedürfnis, eine möglichst hohe Konzentration von aktiven Ribozymen in der Zelle zu gewährleisten, wofür der Transport in die Zelle einer der limitierenden Faktoren ist.

Bedarf an einem effizienten System für das Einführen von Nukleinsäure in lebende Zellen besteht außerdem im Rahmen der Gentherapie. Dabei werden Gene in Zellen eingeschleust, um in vivo die Synthese therapeutisch wirksamer Genprodukte zu erzielen.

Zunehmend besteht Bedarf an Behandlungsmethoden, bei denen die therapeutisch wirksame DNA (oder auch mRNA) wie ein Medikament ("Gentherapeutikum") je nach Erfordernis einmalig oder wiederholt verabreicht wird. Beispiele für genetisch bedingte Erkrankungen, bei denen die Gentherapie einen erfolgversprechenden Ansatz darstellt, sind Hämophilie, beta-Thalassämie und "Severe Combined Immune Deficiency"(SCID), ein Syndrom, das durch einen genetisch bedingten Mangel des Enzyms Adenosindeaminase hervorgerufen wird. Anwendungsmöglichkeiten bestehen weiters bei der Immunregulation, wobei durch Verabreichung funktioneller Nukleinsäure, die für ein sekretiertes Protein-Antigen oder für ein nicht-sezerniertes Protein-Antigen kodiert, mittels einer Impfung eine humorale oder intrazelluläre Immunität erzielt wird. Weitere Beispiele für genetische Defekte, bei denen eine Nukleinsäure, die für das defekte Gen kodiert, z.B. in individuell auf den Bedarf abgestimmter Form, verabreicht werden kann, sind Muskeldystrophie (Dystrophin-Gen), Cystische Fibrose ("Cystic fibrosis transmembrane conductance regulator gene"), Hypercholesterolämie (LDL-Rezeptor-Gen). Gentherapeutische Behandlungsmethoden sind weiters potentiell dann von Bedeutung, wenn Hormone, Wachstumsfaktoren oder cytotoxisch oder immunmodulierend wirkende Proteine im Organismus synthetisiert werden sollen.

Es wurden bereits mehrere Lösungen vorgeschlagen, den Transport von Nukleinsäuren in lebende Zellen, der bei deren therapeutischer Anwendung einen der limitierenden Faktoren darstellt, zu verbessern.

Einer dieser Lösungswege besteht in direkten Modifikationen der Nukleinsäuren, z.B. durch Substitution der geladenen Phosphodiestergruppen durch ungeladene Gruppen. Eine weitere Möglichkeit der direkten Modifikation besteht in der Verwendung von Nukleosidanalogen. Diese Vorschläge weisen jedoch verschiedene Nachteile auf, z.B. geringere Bindung an das Zielmolekül, geringere Hemmwirkung, mögliche Toxizität.

Ein alternativer Ansatz zur direkten Modifikation der Oligonukleotide besteht darin, das Oligonukleotid als solches unverändert zu belassen und mit einer Gruppe zu versehen, die ihm die angestrebten Eigenschaften verleiht, z.B. mit Molekülen, die den Transport in die Zelle erleichtern.

Für die Gentransformation von Säugetierzellen in vitro sind verschiedene Techniken bekannt, deren Anwendbarkeit in vivo jedoch beschränkt ist (dazu zählen das Einbringen von DNA mittels Viren, Liposomen, Elektroporation, Mikroinjektion, Zellfusion, DEAE-Dextran oder die Calciumphosphat-Präzipitationsmethode).

Es wurde daher bereits versucht, ein in vivo anwendbares lösliches System zu entwickeln, das DNA zielgerichtet in die Zellen befördert (Wu und Wu, 1987). Dieses System wurde für Hepatozyten entwickelt und beruht auf dem Prinzip, Polylysin an ein Glykoprotein, auf das ein an der Hepatozytenoberfläche vorhandener Rezeptor anspricht, zu koppeln und daraufhin durch Zugabe von DNA einen löslichen Glykoprotein/Polylysin/DNA-Komplex zu bilden, der in die Zelle aufgenommen wird und nach Aufnahme des Komplexes in die Zelle die Expression der DNA-Sequenz ermöglicht.

Dieses System ist spezifisch für Hepatozyten und wird hinsichtlich seiner Funktion durch den relativ gut charakterisierten Aufnahmemechanismus über den Asialoglykoproteinrezeptor definiert.

Ein breiter anwendbares, effizientes Transportsystem benutzt den Transferrinrezeptor für die Aufnahme von Nukleinsäuren in die Zelle mittels Transferrin-Polykation-Konjugaten. Dieses System ist Gegenstand der EP-A1 0388 758, auf deren Offenbarung hiermit Bezug genommen wird.

Es konnte gezeigt werden, daß mittels dieses Systems in die Zelle transportierte DNA exprimiert wird und daß im Falle der Verwendung inhibierend wirkender Nukleinsäure die inhibierende Wirkung durch das Transportsystem nicht beeinträchtigt wird. Auf dieses System wird im folgenden mit der Bezeichnung "Transferrinfektion" Bezug genommen.

Die Internationale Patentanmeldung WO 91/17773, die eine Veröffentlichung im Sinne des Artikels 54(3) EPÜ darstellt, bezieht sich auf ein System für den Transport von Nukleinsäuren mit spezifischer Wirkung für CD4 exprimierende Zellen. Dieses System macht Gebrauch von dem vom HIV-Virus benutzten Rezeptor, indem die zu importierende Nukleinsäure mit einem Protein-Polykation-Konjugat komplexiert wird, dessen Proteinanteil ein Protein mit der Fähigkeit ist, an CD4 zu binden, und CD4 exprimierende Zellen mit den erhaltenen Protein-Polykation/Nukleinsäure-Komplexen in Berührung gebracht werden. Es konnte nachgewiesen werden, daß mit Hilfe dieses Systems (im folgenden als CD4-Transfektion bezeichnet) in die Zelle transportierte DNA in der Zelle exprimiert wird.

Diesen beiden Erfindungen gemeinsam ist das Merkmal, daß sie spezifische Zellfunktionen benutzen, um den Import der Nukleinsäure in die Zelle zu ermöglichen oder zu erleichtern.

In beiden Fällen laufen die Aufnahmemechanismen unter Beteiligung von Faktoren ab, die im Rahmen der vorliegenden Erfindung als "Internalisierungsfaktoren" bzw. "Liganden", die nach Bindung an die Zielzellen über Fusion mit der Zellmembran internalisiert werden, bezeichnet werden. Darunter sind Liganden zu verstehen, die, im engeren oder weiteren Sinn zelltypspezifisch, gegebenenfalls unter Mitwirkung weiterer Faktoren (z.B. Zelloberflächenproteinen), an die Zelloberfläche binden und internalisiert werden, wobei ihre Internalisierung reversibel oder irreversibel sein kann. Der Internalisierungsfaktor ist mit einer Substanz polykationischen Charakters konjugiert, die aufgrund ihrer Affinität zu Nukleinsäuren zwischen ihr und der Nukleinsäure eine Verbindung herstellt. (Diese Substanzen werden im folgenden als 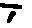 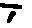 an Nukleinsäure bindende Substanzen" oder "Nukleinsäure-affine Substanzen" bezeichnet.)

Im Zuge der beiden der vorliegenden Erfindung vorangegangenen Erfindungen war festgestellt worden, daß ein Optimum für den Import von Nukleinsäure in die Zelle erzielt werden konnte, wenn das Verhältnis Konjugat:Nukleinsäure so gewählt wurde, daß die Internalisierungsfaktor-Polykation/Nukleinsäure-Komplexe weitgehend elektroneutral waren.

Der vorliegenden Erfindung lag die Aufgabe zugrunde, die Effizienz von Systemen zu verbessern, bei denen die Aufnahme von Nukleinsäuren mittels Internalisierungsfaktoren erfolgt.

Um die gestellte Aufgabe zu lösen, wurde die Zusammensetzung derjenigen Internalisierungsfaktor-Polykation-Nukleinsäure/Komplexe, die hinsichtlich ihrer Aufnahmeeffizienz die besten Ergebnisse gebracht hatten, im Detail untersucht, um die daraus gewonnenen Erkenntnisse für die Optimierung des Systems zu nutzen.

Zunächst wurden verschiedene Transferrin-Polykation-Konjugate synthetisiert (abgesehen von der Isolierungsvorschrift im wesentlichen gemäß der in der EP-A1 0388 758 beschriebenen Herstellungsvorschrift). Jedes Konjugat wurde zunächst in Vorversuchen durch Titration auf seine Fähigkeit untersucht, ein Gen (verwendet wurde das Luciferase-Reportergen) in lebende Zellen zu importieren. Dabei wurde festgestellt, daß jeder Konjugat/DNA-Komplex ein eindeutiges Optimum hinsichtlich des Konjugat/DNA-Verhältnisses aufwies. Es wurde beobachtet, daß Transferrin-Polylysin-Konjugate (TfpL) mit einem hohen Polylysingehalt beim Transport von DNA wesentlich bessere Ergebnisse zeigten als Konjugate mit niedrigerem Polylysingehalt. Für Polylysin 450 (pL 450) wurden vergleichbare Ergebnisse wie für Polylysin 200 (pL 200) erhalten, wobei jedoch ab einem bestimmten pL-Gehalt der Konjugate (Tf/pL 1/1) die Aktivität wieder abnahm. Die besten Ergebnisse wurden bei ca. 100 bis 200 positiv geladenen Aminosäuren pro Transferrin im Konjugat erhalten.

Eine weitere Versuchsreihe wurde nach dem Prinzip durchgeführt, bei Bekanntheit des Konjugat/DNA-Optimums eine suboptimale Menge des jeweiligen Konjugats zu verwenden, dieses mit steigenden Mengen von freiem (nicht kovalent gebundenem) Polykation bzw. einer Substanz polykationischen Charakters (es wurden Polylysine verschiedenen Polymerisationsgrades sowie Protamine und die Histone H1, H3 und H4 verwendet) zu mischen und die erhaltene Mischung einer konstanten DNA-Menge zuzufügen. Die Transfektionseffizienz der Komplexe, die bei der suboptimalen Konjugat-Menge beträchtlich unter dem Transfektionsoptimum lag, konnte durch Zugabe von freiem Polykation vollständig wiederhergestellt oder sogar übertroffen werden. Lediglich Spermidin und Spermin, von denen bekannt war, daß sie nur bei niedriger Ionenstärke DNA kondensieren (Tikchonenko et al., 1988), waren nicht in der Lage, unter den für das Gewebekultursystem physiologischen Bedingungen die Expression von DNA zu steigern.

Im Zuge der im Rahmen der vorliegenden Erfindung durchgeführten Versuche wurde weiters eine Reihe von Transferrin-Protamin-Konjugaten hergestellt und auf ihre Fähigkeit zum Transport von Nukleinsäure in lebende Zellen untersucht, wobei authentische oder synthetische Protamine verwendet wurden. Die untersuchten Konjugate zeigten zwar die Fähigkeit, DNA in die Zelle zu transportieren, ihre Effizienz betrug jedoch nur ca. 1/10 der Effizienz der Polylysin-Konjugate. Mit Hilfe von freiem Polylysin konnte die Effizienz der Protamin-Konjugate wesentlich gesteigert werden.

Übereinstimmende Ergebnisse wurden für das CD4-Transfektionssystem gefunden. Es wurden Versuche mit einem gp120-Polylysin/DNA-Komplex durchgeführt, bei denen von gp120-Polylysin-Konjugaten ausgegangen wurde, die bei einem Konjugat/DNA-Verhältnis von 2:1 relativ schlechte Ergebnisse gezeigt hatten. Der Zusatz von freiem Polylysin resultierte in einer vielfachen Zunahme der Expression des importierten Fremdgens in CD4 exprimierenden Hela-Zellen.

Die erhaltenen Ergebnisse haben zu der Annahme geführt, daß das im Konjugat enthaltene organische Polykation neben seiner Funktion, die Verbindung zwischen Nukleinsäure und Transferrin herzustellen, bei der Aufnahme der Nukleinsäure in die Zelle bzw. bei der Expression eine zusätzliche Rolle spielt.

Im Anschluß an die Titrationsversuche zur Bestimmung der optimalen Transferrin/Polykation-Verhältnisse wurden Untersuchungen zum molekularen Zustand der Komplexe, die mit als optimal ermittelten DNA/Konjugat-Verhältnissen hergestellt worden waren, durchgeführt. Ziel dieser Untersuchungen war es zu prüfen, ob die Änderung der Konjugations-Verhältnisse die höhere Ordnungsstruktur der Komplexe beeinflußt. Zu diesem Zweck wurden Transferrin-Polykation-Plasmid-DNA-Komplexe, hergestellt in analoger Weise wie für die Transferrinfektion, elektronenmikroskopisch untersucht. Die elektronenmikroskopischen Analysen ergaben überraschenderweise, daß die Plasmid-DNA in Gegenwart der Konjugate zu toroiden Strukturen (ähnlich "doughnuts") mit einem Durchmesser von ca. 80 - 100 nm verdichtet vorliegt, und zwar unabhängig von der elektronenmikroskopischen Präparationsmethode. Das Überraschende an diesen Ergebnissen war darin gelegen, daß die "doughnut"-Strukturen, erhalten mit denjenigen Konjugaten, die sich bei der Transferrinfektion als am effizientesten herausgestellt hatten, mit der Struktur von Komplexen zwischen DNA und freiem Polylysin übereinstimmmten, daß also die Fähigkeit von Polylysin, DNA zu kondensieren, nicht dadurch beeinträchtigt wird, daß es an den Internalisierungsfaktor gekoppelt vorliegt. Aus der Literatur war die Kondensation von λ-DNA durch Polylysin bei hoher Salzkonzentration (Laemmli, 1975) bzw. durch Spermidin bei sehr niedriger Ionenstärke (Chattoraj et al., 1978) bekannt.

Im Zuge der Versuche wurde auch festgestellt, daß die durch nicht-kovalent gebundenes organisches Polykation erzielte Steigerung der Expression in einigen Fällen offensichtlich nicht bzw. nicht ausschließlich auf dessen kondensierende Wirkung, sondern auf andere Mechanismen zurückzuführen sein dürfte. Dies wurde im Fall von Histon H4 festgestellt, das eine beträchtliche Steigerung der Transferrinfektionseffizienz bewirkte, ohne daß elektronenmikroskopisch eine auf H4 zurückzuführende Kompaktierung der DNA beobachtet wurde.

Aus den Ergebnissen der durchgeführten Untersuchungen wurde zunächst abgeleitet, daß wenigstens zwei Voraussetzungen für die Effizienz des Imports der Internalisierungsfaktor-Nukleinsäure-affine Substanz/Nukleinsäure-Komplexe wesentlich sind:
a) Das Vorhandensein einer ausreichenden Menge geeigneter Nukleinsäure-affiner Substanz in der Mischung, um die Bindung des Nukleinsäuremoleküls in einer Form zu gewährleisten, die mit dessen Internalisierung und Expression nicht nur kompatibel ist, sondern diese Vorgänge offensichtlich sogar erleichtert. Eine verbesserte Internalisierung ist, z.B. im Falle von Transferrin und freien organischen Polykationen, die eine Kompaktierung der DNA bewirken, möglicherweise darauf zurückzuführen, daß der Durchmesser der kondensierten "doughnuts" mit der Dimension der "coated pits" übereinstimmt, durch die das an seinen Rezeptor gebundene Transferrin internalisiert wird und deren innerer Durchmesser ca. 100 nm beträgt (Darnell et al., 1989). (Die Kondensierung der Nukleinsäure bewirkt gegebenenfalls neben der erleichterten Internalisierung einen Schutz vor enzymatischem Abbau.)
b) Das Vorhandensein von Internalisierungsfaktor in einer Form und einer Menge, um den Transport der Komplexe (z.B. im Fall von Transferrin über Rezeptor-vermittelte Endozytose) in die Zelle aufrechtzuerhalten, wobei die hohe Anzahl von Internalisierungsfaktor-Molekülen pro "doughnut", die sich bei Verwendung von Konjugaten ergibt, die optimales Konjugat/DNA-Verhältnis aufweisen, nicht erforderlich sein dürfte, um eine effiziente Aufnahme der Komplexe in die Zelle zu ermöglichen.

Die Ergebnisse der durchgeführten Versuche haben zu der Annahme geführt, daß die durch freies organisches Polykation bewirkte Expressionssteigerung offensichtlich nicht oder nicht ausschließlich auf DNA-Kompaktierung zurückzuführen sein muß, sondern gegebenenfalls zusätzlich auf eine Schutzwirkung für die DNA und damit eine Inhibierung des DNA-Abbaus in der Zelle und/oder auf eine positive Beeinflussung der Exponierung der Nukleinsäure gegenüber der Expressionsmaschinerie.

Die Erfindung geht somit von der Beobachtung aus, daß bei Verwendung von Internalisierungsfaktor-Polykation-Konjugaten ein Teil der Konjugate durch nicht kovalentgebundene organische Polykationen (bzw. Substanzen polykationischen Charakters) ersetzt werden kann, sofern nach Ersatz eines Teils der Konjugate durch freies organisches Polykation noch Konjugat in ausreichender Menge und in einer Form vorhanden ist, um das Funktionieren des durch den Internalisierungsfaktor vermittelten Aufnahmemechanismus zu gewährleisten.

Die im Rahmen der vorliegenden Erfindung mit Transferrin-Polykation-Konjugaten und Plasmid-DNA durchgeführten Versuche haben ergeben, daß 10 bis 20 Transferrinmoleküle pro DNA-Molekül erforderlich sind, um den Gen-Transport in die Zelle aufrechtzuerhalten. Wenn aufgrund des Ersatzes der Konjugate durch freies organisches Polykation diese Zahl unterschritten wurde, sank die Menge an importierter DNA drastisch, und zwar auf Werte, die typisch sind für reine Kationen-Importsysteme (Farber et al., 1975).

Ohne auf diese Theorie festgelegt sein zu wollen, könnte es, z.B. im Fall von Transferrin entscheidend sein, daß die Transferrin-Moleküle nicht nur in ausreichender bzw. optimaler Anzahl vorhanden sind, sondern daß diese Moleküle auch für den Rezeptor zugänglich sind, d.h. dem Rezeptor in geeigneter Weise präsentiert werden, um den Ablauf der Rezeptorvermittelten Endozytose zu gewährleisten.

Es wurde überraschend festgestellt, daß die Menge der in die Zelle aufgenommenen Nukleinsäure-Menge nicht verringert wird, wenn ein Teil der Transferrin-Polykation-Konjugate durch nicht-kovalent gebundenes organisches Polykation ersetzt wird; in bestimmten Fällen konnte sogar eine beträchtliche Steigerung der DNA-Aufnahme erreicht werden. Die mit CD4 bindenden Proteinen als Internalisierungsfaktor durchgeführten Versuche brachten ähnliche Ergebnisse.

Weiters wurde festgestellt, daß der Zusatz freier organischer Polykationen auch bei Anwendung anderer Nukleinsäure-affiner Substanzen eine Steigerung der Effizienz des Importsystems bewirkt.

Entsprechende Versuche wurden mit dem interkalierenden Agens Ethidiumdimer als Nukleinsäure-affiner Substanz durchgeführt, wobei Transferrin-Ethidiumdimer-Konjugate eingesetzt wurden. Es konnte auch in diesem System eine Steigerung der Expression der mittels der Konjugate in die Zelle eingebrachten DNA nachgewiesen werden, wenn die DNA/Konjugat-Komplexe freies Polykation enthielten.

Die vorliegende Erfindung betrifft somit Komplexe, die in höhere eukaryotische Zellen aufgenommen werden, enthaltend Nukleinsäure (A), die komplexiert ist mit einem Konjugat aus einem Liganden, der nach Bindung an die Zielzellen über Endozytose oder über Fusion mit der Zellmembran internalisiert wird, und einer an Nukleinsäure bindenden Substanz (B), ausgewählt aus der Gruppe organische Polykationen und interkalierende Substanzen. Die Komplexe sind dadurch gekennzeichnet, daß sie zusätzlich ein oder mehrere nicht-kovalent gebundene organische Polykationen (C), die gegebenenfalls identisch sind mit dem in dem Konjugat enthaltenen Polykation (B), in einer Menge enthalten, durch welche die durch das Konjugat erzielte Aufnahme in die Zelle und/oder die Expression der Nukleinsäure gesteigert wird.

Die Erfindung betrifft in einem weiteren Aspekt ein Verfahren zum Einbringen von Nukleinsäure in höhere eukaryotische Zellen, bei dem die Zellen mit den erfindungsgemäßen Komplexen in Berührung gebracht werden.

Mit Hilfe der vorliegenden Erfindung wird bei zumindest gleicher Transfektions/Expressions-Effizienz eine geringere Menge, bezogen auf die in die Zelle zu importierende Nukleinsäure-Menge, an Konjugat aus Internalisierungsfaktor und Nukleinsäure-affiner Substanz benötigt, was einerseits einen geringeren Synthese-Aufwand bedeutet. Eine geringere Konjugatmenge kann andererseits dann von Vorteil sein, wenn der Effekt vermieden werden soll, daß durch eine größere Anzahl von Internalisierungsfaktor-Molekülen innerhalb eines Komplexes gleich mehrere benachbarte "Andock-Stellen" (z.B. Rezeptoren) besetzt werden und in der Folge für weitere Komplexe nicht mehr verfügbar sein können. Die Anzahl der in den Komplexen enthaltenen Menge an Internalisierungsfaktor auf das erforderliche Minimum zu beschränken, d.h. die Konjugatmenge möglichst gering zu halten und mit freiern organischen Polykation zu verdünnen, ist insbesondere auch dann von Vorteil, wenn die Zahl von Rezeptoren auf den zu behandelnden Zielzellen gering ist.

Mit Hilfe der vorliegenden Erfindung kann die Leistungsfähigkeit von Konjugaten, die für sich weniger effizient sind, beträchtlich erhöht werden und die Leistungsfähigkeit von an sich schon sehr effizienten Konjugaten noch weiter gesteigert werden.

Hinsichtlich der qualitativen Zusammensetzung der erfindungsgemäßen Komplexe werden im allgemeinen zunächst die in die Zelle zu importierende Nukleinsäure und der Ligand festgelegt. Die Nukleinsäure wird vor allem durch den in der Zelle zu erzielenden biologischen Effekt definiert, z.B. durch die Zielsequenz des zu inhibierenden oder - im Falle der Anwendung im Rahmen der Gentherapie - des zur Expression zu bringenden Gens bzw. Genabschnitts, z.B. zwecks Substitution eines defekten Gens. Gegebenenfalls ist die Nukleinsäure modifiziert, bedingt z.B. durch eine für den jeweiligen Anwendungsfall erforderliche Stabilität. Bei den in die Zelle zu transportierenden Nukleinsäuren kann es sich um DNAs oder RNAs handeln, wobei hinsichtlich der Nukleotidsequenz keine Beschränkungen bestehen. Modifikationen können z.B. in der Substitution der Phosphodiestergruppe durch Phosphorothioate oder in der Verwendung von Nukleosidanalogen bestehen (Zon, 1988). Als therapeutisch wirksame inhibierende Nukleinsäuren sind vor allem diejenigen von Interesse, die zum Zweck der Inhibierung spezifischer Gensequenzen in die Zelle transportiert werden sollen. Dazu zählen Antisense-Oligonukleotide und Ribozyme, gegebenenfalls zusammen mit einer Carrier-Nukleinsäure (bzw. Genkonstrukte, von denen Antisense-RNA oder Ribozyme transkribiert werden).

Der Ligand wird vor allem durch die Zielzellen definiert, z.B. durch bestimmte Oberflächenantigene oder Rezeptoren, die für einen Zelltyp spezifisch sind und somit einen gerichteten Import der Nukleinsäure in diesen Zelltyp ermöglichen.

Ausgehend von der Festlegung von Nukleinsäure und Ligand wird die an Nukleinsäure bindende Substanz auf diese Parameter abgestimmt, wobei die Größe der Nukleinsäure, vor allem im Hinblick auf eine weitgehende Neutralisierung der negativen Ladungen, eine wesentliche Rolle spielt.

Für die Wahl der in den Komplexen enthaltenen nicht-kovalent gebundenen organischen Polykationen ist entscheidend, daß durch ihren Zusatz gegenüber der durch die Konjugate erzielbaren Internalisierung/Expression der Nukleinsäure eine Steigerung bewirkt wird.

Ebenso wie die qualitative Zusammensetzung richtet sich auch die quantitative Zusammensetzung der Komplexe nach mehreren Kriterien, die miteinander in funktionellem Zusammenhang stehen, z.B. ob und in welchem Ausmaß es erforderlich oder erwünscht ist, die Nukleinsäure zu kondensieren, welche Ladung der Gesamtkomplex aufweisen soll, in welchem Ausmaß Bindungs- und Internalisierungsfähigkeit für den jeweiligen Zelltyp gegeben ist und in welchem Ausmaß deren Steigerung erwünscht bzw. erforderlich ist. Weitere Parameter für die Zusammensetzung des Komplexes sind die Zugänglichkeit des Liganden für den Rezeptor, wobei dafür die Art maßgeblich ist, wie dieser innerhalb des Komplexes der Zelle gegenüber präsentiert wird. Wesentlich ist weiters die Zugänglichkeit der Nukleinsäure in der Zelle, um deren bestimmungsgemäße Funktion auszuüben.

Unter 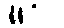 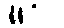 Internalisierungsfaktor" bzw. 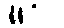 Ligand" sind im Rahmen der vorliegenden Erfindung Liganden oder Fragmente davon zu verstehen, die nach Bindung an die Zelle über Endozytose, vorzugsweise Rezeptor-vermittelte Endozytose, internalisiert werden, oder Faktoren, deren Bindung/Internalisierung über Fusion mit Zellmembranelementen erfolgt.

Zu grundsätzlich geeigneten Internalisierungsfaktoren zählen die Liganden Transferrin, Conalbumin; Asialoglykoproteine (wie Asialotransferrin, Asialorosomucoid oder Asialofetuin) bzw. Substanzen, die Galaktose enthalten und über den Asialoglykoproteinrezeptor internalisiert werden; Lipoproteine, die über Rezeptoren in die Zelle aufgenommen werden (apo B100/LDL); virale Proteine wie das HIV-Protein gp120; Antikörper gegen Oberflächenantigene, z.B. anti-CD4, anti-CD7; Zytokine wie Interleukin-1, TNF; Faktoren und Wachstumsfaktoren wie Insulin, EGF; entwaffnete Toxine. Die Liganden können natürlichen oder synthetischen Ursprungs sowie gegebenenfalls modifiziert sein.

Wesentlich für die Eignung solcher Faktoren im Rahmen der vorliegenden Erfindung ist,
a) daß sie vom spezifischen Zelltyp, in den die Nukleinsäure eingebracht werden soll, internalisiert werden können und ihre Internalisierungsfähigkeit nicht oder nicht wesentlich beeinträchtigt wird, wenn sie mit dem Verbindungsfaktor konjugiert werden, und
b) daß sie in Rahmen dieser Eigenschaft in der Lage sind, Nukleinsäure auf dem von ihnen benutzten Weg in die Zelle "huckepack" mitzunehmen.

Im Rahmen der vorliegenden Erfindung geeignete an Nukleinsäure bindende Substanzen sind z.B. homologe Polykationen wie Polylysin, Polyarginin, Polyornithin oder heterologe Polykationen mit zwei oder mehr unterschiedlichen positiv geladenen Aminosäuren, wobei diese Polykationen verschiedene Kettenlänge aufweisen können, weiters nichtpeptidische synthetische Polykationen wie Polyethylenimin. Geeignete Verbindungsfaktoren sind weiters natürliche DNA bindende Proteine polykationischen Charakters wie Histone oder Protamine bzw. Analoge oder Fragmente davon. Weitere geeignete Verbindungsfaktoren sind interkalierende Substanzen wie Ethidiumdimere, Acridin oder interkalierende Peptide, enthaltend Tryptophan und/oder Tyrosin und/oder Phenylalanin.

Die in nicht-kovalent gebundener Form in den Komplexen enthaltenen organischen Polykationen können gleich oder verschieden sein von denen des Konjugats. Ein wesentliches Kriterium für deren Auswahl ist die Größe der Nukleinsäure, insbesondere im Hinblick auf deren Kondensierung; bei kleineren Nukleinsäure-Molekülen ist im allgemeinen eine Kompaktierung nicht erforderlich. Die Auswahl der freien organischen Polykationen nach Art und Menge wird außerdem auf das Konjugat abgestimmt wobei vor allem die im Konjugat enthaltene Nukleinsäure-affine Substanz zu berücksichtigen ist: ist die Nukleinsäure-affine Substanz z.B eine Substanz, die keine oder nur geringe Fähigkeit zur DNA-Kondensation aufweist, ist es im Hinblick auf eine effiziente Internalisierung der Komplexe im allgemeinen zweckmäßig, solche organische Polykationen einzusetzen, die diese Eigenschaft in stärker ausgeprägtem Maß besitzen. Hat die Nukleinsäure-affine Substanz selbst die Fähigkeit, Nukleinsäure zu kondensieren und wird bereits durch sie eine im Hinblick auf effiziente Internalisierung ausreichende Kompaktierung der Nukleinsäure bewirkt, wird zweckmäßigerweise ein organisches Polykation verwendet, das aufgrund anderer Mechanismen eine Steigerung der Expression bewirkt.

Zu den im Rahmen der vorliegenden Erfindung geeigneten nicht-kovalent gebundenen organischen Polykationen zählen Verbindungen mit der Fähigkeit, Nukleinsäure zu kondensieren und/oder diese vor unerwünschtem Abbau in der Zelle zu schützen. Eine weitere Gruppe geeigneter Substanzen sind solche, die durch Bindung an die Nukleinsäure eine Verbesserung deren Transkription/Expression bewirken, indem sie die Zugänglichkeit der Nukleinsäure für die Expressionsmaschinerie der Zelle verbessern. Ein Beispiel für eine solche Substanz ist das chromosomale Nicht-Histon-Protein HMGI, von dem festgestellt wurde, daß es die Fähigkeit aufweist, DNA zu kompaktieren und in der Zelle zur Expression zu bringen, wobei angenommen wird, daß eine Aktivierung der DNA für die Transkription stattfindet (Böttger et al., 1988).

Die an Nukleinsäure bindenden Substanzen und/oder die nicht-kovalent gebundenen organischen Polykationen sind gegebenenfalls modifiziert. Die Modifizierung kann bestehen in lipophilen Gruppierungen, z.B. Kohlenwasserstoffgruppierungen mit Ähnlichkeit zu natürlichen Lipiden (z.B. Fettsäuren, Cholesterin), die geeignet sind, die Affinität der Komplexe zur Zellmembran zur erhöhen.

Eine weitere Möglichkeit zur Modifikation besteht in hydrophilen Gruppierungen, die geeignet sind, die Spezifizät der Komplexe für die Zielzellen zu erhöhen, indem durch solche Gruppierungen verhindert wird, daß die Komplexe zu anderen Zellen, die Affinität zu lipophilen Gruppierungen aufweisen, fehlgeleitet werden. Beispiele für solche Gruppierungen sind Zucker, wie Laktose, Maltose, Galaktose, sowie Polyethylenglykol.

Bei der Bestimmung des molaren Verhältnisses Ligand/Nukleinsäure-affine Substanz innerhalb der Konjugate ist zu berücksichtigen, daß Komplexierung der Nukleinsäure(n) stattfindet und gewährleistet ist, daß der gebildete Komplex an die Zelle gebunden und in die Zelle befördert wird. Die Konjugate können durch einfach durchzuführende Vorversuche, in denen Konjugate unterschiedlicher Zusammensetzung auf ihre Effizienz untersucht werden, definiert werden.

Das jeweils gewählte Verhältnis richtet sich vor allem nach der Größe des Polykationmoleküls sowie nach der Anzahl und Verteilung der positiv geladenen Gruppierungen, Kriterien, die auf Größe, Struktur sowie allenfalls vorhandene Modifikationen der zu transportierenden Nukleinsäure(n) abgestimmt werden.

Nach Konstruktion und Synthese der Konjugate und der Bestimmung des für die Effizienz der Transferrinfektion optimalen Verhältnisses Konjugat:DNA kann mit Hilfe von Titrationen die Menge des Konjugat-Anteils bestimmt werden, der durch freies organisches Polykation ersetzbar ist.

Die erfindungsgemäßen Komplexe können hergestellt werden, indem die Komponenten Nukleinsäure, Internalisierungsfaktor/Nukleinsäure-affine Substanz-Konjugat und freies Polykation, die jeweils in Form verdünnter Lösungen vorliegen, gemischt werden. Im Falle der Verwendung von Polykationen auch als Nukleinsäure-affine Substanz ist es im allgemeinen zweckmäßig, zuerst eine Mischung von Konjugaten mit freien Polykationen herzustellen und diese Mischung mit DNA zu vereinigen. Dabei wird das optimale Verhältnis von DNA zur jeweiligen Konjugat-Polykationenmischung durch Titrationsexperimente, d.h. in einer Reihe von Transfektionsexperimenten mit konstanter DNA-Menge und zunehmender Menge an Konjugat-Polykationenmischung, bestimme. Das optimale Verhältnis von Konjugat : Polykationen in der Mischung ergibt sich aus der Anwendung bzw. aus dem Vergleich der Optima der in den Titrationsexperimenten eingesetzten Mischungen.

Die Herstellung der DNA-Komplexe kann bei physiologischen Salzkonzentrationen erfolgen. Eine weitere Möglichkeit besteht aber auch im Einsatz hoher Salzkonzentrationen (etwa 2M NaCl) und anschließende Einstellung auf physiologische Bedingungen durch langsames Verdünnen bzw. Dialyse.

Die für die spezielle Ausführungsform der Erfindung jeweils am besten geeignete Reihenfolge für die Mischung der Komponenten Nukleinsäure, Konjugat, nicht-kovalent gebundenes organisches Polykation wird im einzelnen in Vorversuchen bestimmt. Fallweise kann es sich als zweckmäßig erweisen, zuerst die Nukleinsäure mit dem Konjugat zu komplexieren und erst dann das freie organische Polykation zuzufügen, z.B. im Fall von Transferrin-Ethidiumdimer-Konjugaten und Polylysin.

In einer Ausführungsform der Erfindung ist der Ligand Transferrin und die an Nukleinsäure bindende Substanz ein Polykation. Die Aufnahme der Nukleinsäure erfolgt in Form von Komplexen, in denen Transferrin-Polykation-Konjugate mit Nukleinsäure komplexiert sind, wobei das Polykation in nicht kovalent gebundener Form in den Komplexen enthalten ist und das freie organische Polykation gleich oder verschieden ist von dem im Konjugat enthaltenen Polykation.

Unter "Transferrin" sind sowohl die natürlichen Transferrine zu verstehen als auch diejenigen Transferrin-Modifikationen, die vom Rezeptor gebunden und in die Zelle transportiert werden.

Bei der Bestimmung des molaren Verhältnisses Transferrin:Polykation innerhalb der Konjugate ist zu berücksichtigen, daß Komplexierung der Nukleinsäure(n) stattfindet und gewährleistet ist, daß der gebildete Komplex durch den Transferrinrezeptor gebunden und in die Zelle befördert wird, was durch einfach durchzuführende Versuche von Fall zu Fall überprüft werden kann.

Als kationische Substanzen innerhalb der Konjugate können folgende Verbindungen verwendet werden:
a) Protamine: Diese können natürlichen Ursprungs oder auf rekombinantem Weg hergestellt sein, wobei Mehrfachkopien hergestellt bzw. Modifikationen hinsichtlich Molekülgröße und Aminosäuresequenz vorgenommen werden können. Entsprechende Verbindungen können auch chemisch synthetisiert werden. Bei der Synthese eines künstlichen Protamins kann z.B. so vorgegangen werden, daß Aminosäurereste, die beim natürlichen Protamin Funktionen haben, die für die Transportfunktion unerwünscht sind (z.B. Kondensation von DNA zu größeren Aggregaten) durch geeignete andere Aminosäuren ersetzt werden und/oder an einem Ende eine Aminosäure (z.B. Cystein) vorgesehen wird, die die gezielte Konjugation mit Transferrin ermöglicht.
b) Histone: Es können natürliche und synthetische Histone bzw. Fragmente davon verwendet werden, wobei bezüglich der Herstellung und der Modifikationen grundsätzlich das für Protamine Gesagte gilt.
c) Synthetische Polypeptide, wie homologe Polypeptide (Polylysin, Polyarginin) oder heterologe Polypeptide (bestehend aus zwei oder mehr Vertretern positiv geladener Aminosäuren).
d) Nichtpeptische Polykationen wie Polyethylenimine.

Die Größe der Polykationen richtet sich in erster Linie nach der zu transportierenden Nukleinsäure.

Die Transferrin-Polykation-Konjugate können auf chemischem oder, falls das Polykation ein Polypeptid ist, auf rekombinantem Weg hergestellt werden.

Die Kopplung auf chemischem Weg kann in für die Kopplung von Peptiden an sich bekannter Weise erfolgen, wobei, falls erforderlich, die Einzelkomponenten vor der Kopplungsreaktion mit Linkersubstanzen versehen werden (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird.

Je nach gewünschten Eigenschaften der Konjugate, insbesondere im Hinblick auf deren Stabilität, kann die Kopplung erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

Die Herstellung der Konjugate auf rekombinantem Weg bietet den Vorteil, genau definierte, einheitliche Verbindungen erhalten zu können, während bei der chemischen Kopplung Konjugat-Gemische entstehen, die aufgetrennt werden müssen. Für die Herstellung auf rekombinantem Weg kommen vor allem Protamin- und Histon-Konjugate in Betracht.

Die rekombinante Herstellung der Konjugate ist nach für die Herstellung von chimären Polypeptiden bekannten Methoden durchführbar. Dabei können die polykationischen Peptide hinsichtlich ihrer Größe und Aminosäuresequenz variiert werden. Die gentechnologische Herstellung bietet auch den Vorteil, den Transferrin-Anteil des Konjugats modifizieren zu können, indem z.B. durch geeignete Mutationen die Bindungsfähigkeit an den Rezeptor gesteigert werden kann oder indem der Transferrinanteil auf den für die Bindung an den Rezeptor maßgeblichen Teil des Moleküls verkürzt wird.

Wenn der Ligand ein Glykoprotein ist, z.B. Transferrin, kann er mit der Nukleinsäure-affinen Substanz über eine oder mehrere Kohlenhydratketten des Glykoproteins miteinander verbunden sein.

Solche Konjugate haben den Vorteil, daß sie frei von Modifikationen sind, die von den verwendeten Linkersubstanzen stammen. Diese Konjugate weisen im Falle von Glykoproteinen, die nur eine oder wenige für die Kopplung geeignete Kohlenhydratgruppen aufweisen, z.B. Transferrin, außerdem den Vorteil auf, daß sie hinsichtlich ihrer Bindungsstelle Glykoprotein/Verbindungsfaktor exakt definiert sind.

Eine geeignete Methode zur Herstellung von Glykoprotein-Polykation-Konjugaten ist in der deutschen Patentanmeldung P 41 15 038.4 geoffenbart; sie wurde von Wagner et al., 1991, beschrieben.

Die vorliegende Erfindung bietet u.a. den Vorteil, die Effizienz von Transferrin-Protamin-Konjugaten, die zwar für sich weniger leistungsfähig als Polylysin-Konjugate, jedoch auf rekombinantem Weg unter Erzielung von Konjugaten definierter Zusammensetzung relativ einfach herstellbar sind, zu steigern. Bezüglich der verwendbaren Transferrin-Polykation-Konjugate bzw. der Komplexe mit DNA sowie deren Herstellung im einzelnen wird auf die Offenbarung der EP-A1 0388 758 Bezug genommen.

Die obigen Angaben zeigen anhand von Transferrin-Komplexen mögliche Ausführungsformen der Erfindung, sind jedoch ausdrücklich nicht auf Transferrin-Komplexe beschränkt.

Für die Bestimmung geeigneter Transfektionsbedingungen wird - beispielhaft illustriert anhand der Verwendung von Transferrin-Polykation-Konjugaten - zweckmäßigerweise wie folgt vorgegangen: Ausgehend von einer durch die Anwendung (Transfer von Genen oder Genabschnitten, Innibierung von Zellfunktionen durch Antisense-Oligonukleotide, etc.) definierten, in die Zelle zu transportierenden Nukleinsäure werden Transferrin-Polykation-Konjugate synthetisiert, die mit der Nukleinsäure komplexiert und über den Transferrin-Rezeptor in die Zelle aufgenommen werden können. In dem jeweiligen Zellsystem, in dem die Transfektion erfolgen soll, werden zunächst die geeigneten Transfektionsbedingungen gewählt, insbesondere im Hinblick auf die zu transfizierenden Zellen bzw. deren Zustand, z.B. Bedingungen, die eine Erhöhung der Transferrin-Rezeptorzahl bewirken und/oder den Abbau der Nukleinsäure in Zellorganellen, insbesondere Lysosomen, inhibieren.

Dabei ist nicht entscheidend, ob die Erhöhung der Aufnahme von Transferrin-Polykation/Nukleinsäure-Komplexen ausschließlich auf die Erhöhung der Transferrin-Rezeptor-Anzahl mittels direkter oder indirekter Einwirkung auf den Rezeptor (Steigerung der Rezeptor-Synthese, z.B durch Steigerung der Transkriptionsrate, Inhibierung des mRNA-Abbaus oder erhöhte Translationsrate der mRNA, Verminderung des Rezeptorabbaus oder Kombinationen solcher Mechanismen) zurückzuführen ist. Es können an diesem Effekt noch andere Mechanismen, z.B eine Erhöhung der Affinität von Transferrin an seinen Rezeptor und/oder eine Erhöhung der Recyclingrate des Transferrin-Rezeptors und/oder die Inhibierung der Kompetition von nativem Transferrin mit den Transferrin-Polykation/Nukleinsäure-Komplexen um die Bindung an den Transferrin-Rezeptor, beteiligt sein. Entscheidend ist, daß die Bedingungen, denen die Zellen ausgesetzt werden, in einer Erhöhung der Aufnahme der Transferrin-Polykation-Nukleinsäure-Komplexe resultieren. Unter der Definition "Bedingungen, die eine Erhöhung der Transferrin-Rezeptor-Anzahl bewirken" sind somit auch Bedingungen mitumfaßt, die eine Steigerung der Effizienz der Transferrinfektion aufgrund der oben angeführten Mechanismen bewirken.

Geeignete Bedingungen, denen die Zellen ausgesetzt werden, um die Transferrin-Rezeptormenge zu erhöhen, bestehen z.B. darin, die Zelle mit Substanzen in Berührung zu bringen, die geeignet sind, die Hämkonzentration im Inneren der Zelle zu senken.

Dabei handelt es sich bevorzugt um Substanzen, die eine Verringerung der Hämkonzentration in der Zelle bewirken, indem sie
a) die Protoporphyrin IX-Biosynthese hemmen
b) die Hämbildung verhindern
c) den Hämabbau fördern.

Zu den Substanzen der Gruppe a) zählt Succinylaceton, wobei die Erhöhung der Transferrin-Rezeptorzahl wahrscheinlich auf die Inhibierung der Protoporphyrin IX-Biosynthese zurückzuführen ist.
Zu den Substanzen der Gruppe b) zählen diejenigen, die einen Eisenmangel in der Zelle induzieren, z.B.der Eisenchelatbildner Desferrioxamin, der vermutlich eine Erhöhung der Transferrin-Rezeptorzahl bewirkt, weil die Umwandlung von Protoporphyrin IX in Häm verhindert wird. Grundsätzlich sind all die Substanzen, insbesondere Eisenchelatbildner, die in die Zelle aufgenommen werden können und die für Desferrioxamin festgestellte Wirkung auf die für die Hämbildung verfügbare Eisenmenge haben, zur Erhöhung der Transferrinrezeptorzahl geeignet.

Der angeführten Substanzgruppe kommt für die Anwendung von Transferrin-Komplexen im Rahmen der vorliegenden Erfindung weiters insofern Bedeutung zu, als sie offensichtlich geeignet sind, die Kompetition von nativem Transferrin mit den Transferrin-Polykation/Nukleinsäure-Komplexen um die Bindung an den Rezeptor aufzuheben. Durch die Vorbehandlung von Zellen - in vivo oder in vitro - mit solchen Substanzen kann das nicht Transferrin-gebundene Eisen entfernt werden, womit kein Eisen mehr für das aus der Zelle ausgeschleuste Transferrin verfügbar ist und dieses somit als Apotransferrin nicht mit den Transferrin-Polykation/Nukleinsäure-Komplexen um die Bindung an den Transferrin-Rezeptor konkurriert. Durch die Entfernung von freiem Eisen kann andererseits eine Senkung der Transferrin-Rezeptorzahl durch Anwesenheit von freiem Eisen verhindert werden.

Zur Gruppe c) zählen Substanzen, die den Hämabbau induzieren bzw. stimulieren, worauf die Zelle ebenfalls durch Erhöhung der Transferrin-Rezeptor-Konzentration bzw. der Affinität von Transferrin zu seinem Rezeptor bzw. der Rezeptor-Recycling-Rate.
(Es wurde festgestellt, daß mit Hilfe dieser Substanzen die Aufnahme von DNA in die Zelle verbessert werden kann, wobei Kombinationen von Substanzen der Gruppen a) bis c) einen additiven Effekt, bestimmt über die Aktivität des exprimierten Reportergens, aufwiesen.)

Im Rahmen der vorliegenden Erfindung kann es weiters zweckmäßig sein, Bedingungen zu schaffen, unter denen der Abbau der Nukleinsäure in der Zelle inhibiert wird.

Solche Bedingungen können durch Zusatz sog. lysosomatroper Substanzen geschaffen werden. Von diesen Substanzen ist bekannt, daß sie die Aktivität von Proteasen und Nukleasen in Lysosomen hemmen und somit den Abbau von Nukleinsäuren verhindern können, wobei durch die Definition der Eigenschaften lysosomatroper Substanzen auch deren potentielle Fähigkeit miteingeschlossen ist, die Freisetzung von Nukleinsäure aus Zellorganellmembranen innerhalb der Zelle zu ermöglichen bzw. zu erleichtern.

Zu diesen Substanzen zählen Chloroquin, Monensin, Nigericin, Ammoniumchlorid und Methylamin.

Die Notwendigkeit für die Anwendung einer Substanz aus der Gruppe der lysosomatropen Substanzen richtet sich vor allem nach dem zu behandelnden Zelltyp. Die Eignung einer Substanz aus dieser Gruppe im Rahmen der vorliegenden Erfindung sowie deren Konzentration bzw. Einwirkzeit auf die Zellen richtet sich einerseits nach deren Toxizität für die Zellen, andererseits z.B. auch danach, ob diese Substanzen das Cycling des Transferrinrezeptors beeinflussen, etwa durch Blockierung der Vesikelfusion und dadurch Verhinderung der Ausschleusung des Rezeptors.
Bezüglich der Verwendung von den Nukleinsäureabbau inhibierenden Substanzen wurde festgestellt, daß sie nicht bei allen Zelltypen für eine effiziente Transferrinfektion erforderlich ist.

Bei der Anwendung von lysosomatropen Substanzen ist es daher notwendig, deren Erfordernis bzw. Eignung zur Verstärkung der Transferrinfektion in Vorversuchen zu testen. Gegebenenfalls können diese Substanzen bei kürzerer Einwirkzeit schubweise zur Anwendung kommen, um eine etwa die Zelle schädigende Wirkung auszuschalten bzw. möglichst gering zu halten.

In einer weiteren Ausführungsform der vorliegenden Erfindung ist der Ligand ein Protein mit der Fähigkeit, an CD4 zu binden ("CD4 bindendes Protein", "CD4BP"). Es wurde im Rahmen der in der WO 91/17773 geoffenbarten Erfindung festgestellt, daß der vom HIV-Virus bei der Infektion benutzte Rezeptor, CD4, für den Transport von Nukleinsäuren in die Zelle ausgenutzt werden kann, indem die in die Zelle zu importierende Nukleinsäure mit einem Protein-Polykation-Konjugat komplexiert wird, dessen Proteinanteil ein CD4 bindendes Protein ist.
Als CD4BPs können alle diejenigen monoklonalen Antikörper gegen CD4 bzw. Fragmente davon, die an CD4 binden, verwendet werden, z.B. Fab'-Fragmente (Pelchen-Matthews et al., 1989).

Dazu zählen monoklonale Anti-CD4-Antikörper, die ein gp120-Epitop aufweisen, das mit dem Virus um die Bindung an dieses Epitop zu konkurriert.

Anstelle konventioneller monoklonaler Antikörper bzw. deren Fragmente können CD4-Antigen bindende Antikörperabschnitte, bestehend aus einer Kombination von Segmenten der schweren und leichten Kette oder gegebenenfalls der schweren Kette allein, verwendet werden. Als CD4BPs kommen weiters HIV-1-gp120 bzw. homologe Proteine verwandter Retroviren bzw. Fragmente davon in Betracht. Geeignete gp120-Fragmente sind diejenigen, die zur Bindung an CD4 befähigt sind (Lasky et al., 1987), z.B die 95-kDa und 25-kDa-Fragmente, von denen nachgewiesen wurde, daß sie an CD4 binden (Nygren et al., 1988). Solche Fragmente können z.B erhalten werden, indem entweder zunächst das gesamte gp120 Protein auf rekombinantem Weg hergestellt und anschließend proteolytisch gespalten wird. Eine alternative Methode besteht in der rekombinanten Herstellung der Fragmente selbst.

Für den Polykationenanteil der Konjugate bzw. für die nicht-kovalent gebundenen organischen Polykationen gilt prinzipiell das für die Transferrin-Polykation-Konjugate Gesagte.

Bezüglich des molaren Verhältnisses CD4BP:Polykation innerhalb der Konjugate sowie bezüglich der Erfordernisse Komplexierung der Nukleinsäure(n), Bindung durch CD4 und Beförderung in die Zelle gelten grundsätzlich die oben für die Verwendung von Transferrin-Polykation-Konjugaten angeführten Überlegungen. Mit Hilfe von CD4 exprimierenden Zellinien, die mit den Konjugaten in Berührung gebracht werden und die daraufhin auf das Vorhandensein von Nukleinsäure in der Zelle untersucht werden, z.B. durch Southern Blot Analyse, Hybridisierung mit radioaktiv markierten komplementären Nukleinsäuremolekülen, durch Amplifikation mit Hilfe der PCR oder durch Nachweis des Genproduktes eines Reportergens, kann die Leistungsfähigkeit der Komplexe überprüft werden.
Nach Bestimmung des für die Effizienz der Transferrinfektion optimalen Verhältnisses CD4BP-Polykation:DNA kann, analog der Vorgangsweise bei Verwendung von Transferrin-Konjugaten, mit Hilfe von Titrationen die Menge des Konjugat-Anteils bestimmt werden, der durch freies organisches Polykation ersetzbar ist, wobei auch bei dieser Anwendungsform der Erfindung das freie Polykation gegebenenfalls vom Polykationanteil des Konjugats verschieden sein kann.

Auch im Falle der Verwendung von CD4BP-Konjugaten kann es zweckmäßig sein, die Effizienz des Systems durch Einsatz von Substanzen zu verbessern, die den Abbau von Nukleinsäure in der Zelle inhibieren, wobei bzgl. Eignung und Notwendigkeit solcher Maßnahmen grundsätzlich dieselben Überlegungen gelten wie bei der Verwendung von Transferrin-Polykation-Konjugaten.

In den Versuchen, die im Rahmen der vorliegenden Erfindung durchgeführt wurden, wurde als Beispiel für in die Zelle zu transportierende Nukleinsäure Plasmid-DNA, enthaltend das Photinus pyralis Luciferase-Gen, verwendet. Diese DNA ist in der Praxis vor allem für Vorversuche von Bedeutung, die dazu dienen, die im einzelnen, z.B. für einen bestimmten Zelltyp, zu wählenden Bedingungen zu bestimmen.

Bei der therapeutischen Anwendung wird die Nukleinsäure vor allem durch den in der Zelle zu erzielenden biologischen Effekt definiert, im Falle der Anwendung im Rahmen der Gentherapie durch das zur Expression zu bringende Gen bzw. den Genabschnitt, z.B. zwecks Substitution eines defekten Gens, oder durch die Zielsequenz eines zu inhibierenden Gens. Bei den in die Zelle zu transportierenden Nukleinsäuren kann es sich um DNAs oder RNAs handeln, wobei hinsichtlich der Nukleotidsequenz keine Beschränkungen bestehen. Als therapeutisch wirksame inhibierende Nukleinsäuren werden insbesondere Genkonstrukte zum Zweck der Inhibierung spezifischer Gensequenzen in die Zelle transportiert. Dazu zählen Genkonstrukte, von denen Antisense-RNA oder Ribozyme transkribiert werden.

Beispiele für im Rahmen der Gentherapie anwendbare Gene, die mit Hilfe der vorliegenden Erfindung als Bestandteil von Genkonstrukten in die Zelle eingeschleust werden können, sind Faktor IX (Anwendung bei Hämophilie; Kurachi und Davie, 1982), Adenosindeaminase (SCID; Valerio et al., 1984), α-1 Antitrypsin (Lungenemphysem; Ciliberto et al., 1985) oder das "Cystic fibrosis transmembrane conductance regulator gene" (Riordan et al., 1989). Diese Beispiele stellen keinerlei Beschränkung dar.

Bezüglich der Größe der Nukleinsäuren sind Anwendungen in einem weiten Bereich möglich; mit Hilfe der vorliegenden Erfindung können Nukleinsäure-Moleküle einer Größenordnung von ca. 0.3 kb bis ca. 50 kb und darüber in die Zelle befördert werden.

Die Erfindung betrifft in einem weiteren Aspekt pharmazeutische Zubereitungen, enthaltend einen Komplex mit einer ein Gen spezifisch inhibierenden Nukleinsäure als wirksame Komponente. Erfindungsgemäße Komplexe, enthaltend eine derartige inhibierende Nukleinsäure, etwa in Form von Antisense-Oligonukleotiden, Antisense-Oligonukleotidanaloga oder Ribozymen bzw. den dafür kodierenden DNAs, gegebenenfalls zusammen mit einer Carriernukleinsäure wie tRNA, z.B. in Form von Lyophilisaten, können verwendet werden, um im menschlichen oder tierischen Organismus pathogene Viren, wie HIV oder verwandte Retroviren, Oncogene oder andere Schlüsselgene, die das Wachstum und/oder die Differenzierung von Zellen kontrollieren, z.B. das c-fos Gen oder das c-myc Gen, zu inhibieren.

Ein weiteres Anwendungsgebiet der erfindungsgemäßen pharmazeutischen Zubereitungen liegt in der Bekämpfung von Krankheiten durch Hemmung der Produktion von unerwünschten Genprodukten, z.B. des bei der Alzheimer-Krankheit auftretenden Haupt-Plaqueproteins ("major plaque protein") oder Autoimmunerkrankungen verursachender Proteine.

Die Dosierung der erfindungsgemäßen, freies organisches Polykation enthaltenden Internalisierungsfaktor/+ Nukleinsäure-affine Substanz/Nukleinsäure-Komplexe richtet sich vor allem nach der Konzentration der jeweils zu behandelnden Zellen und nach der inhibierenden Nukleinsäure. Die Menge der angewendeten Komplexe hängt z.B. auch davon ab, ob die Nukleinsäure als solche die wirksame Komponente darstellt oder ob sie, z.B. im Fall von Ribozymen, als Gen in die Zelle importiert und in der Zelle amplifiziert wird.

Die für eine bestimmte Indikation beim jeweiligen Zelltyp zur Anwendung gelangende optimale Dosierung wird im einzelnen in Vorversuchen bestimmt.

Für die Therapie kommen in vivo oder ex vivo Anwendungen in Betracht. Eine in vivo Anwendung für die Behandlung von Tumoren kann beispielsweise darin bestehen, eine Lösung eines geeigneten Komplexes, enthaltend z.B. als Nukleinsäure ein gegen ein aktiviertes Oncogen gerichtetes Ribozym, in das Tumorgewebe zu injizieren.

Eine ex vivo Anwendung kommt z.B. für die Behandlung leukämischer Zellen in Betracht. Dabei kann beispielsweise so vorgegangen werden, daß Knochenmark oder peripheres Blut dem Patienten entnommen wird. Daraufhin werden die leukämischen Zellen selektiv unter für die Transferrinfektion optimierten Bedingungen (z.B. nach Vorbehandlung mit Desferrioxamin) mit Transferrin-Polykation/Nukleinsäure-Komplexen behandelt, die als Nukleinsäurekomponente ein die Onkogenaktivität neutralisierendes Anisenseoligonukleotid oder Ribozym enthalten. Abschließend wird das Knochenmark bzw. Blut in den Organismus reimplantiert.

Mit Hilfe der vorliegenden Erfindung ist es möglich, bei therapeutischen Anwendungen von pharmazeutischen Zubereitungen, die Internalisierungsfaktor/Nukleinsäure-affine Substanz/Nukleinsäure-Komplexe enthalten, eine verbesserte Wirkung zu erzielen, indem der Import der wirksamen Nukleinsäure in die Zelle verbessert bzw. deren Expression verstärkt wird.

### Figurenübersicht

- Fig.1A:: Bestimmung des optimalen Verhältnisses von Transferrin-Polylysin-Konjugaten: DNA (Transferrin-Polylysin200)
- Fig.1B:: Bestimmung des optimalen Verhältnisses von Transferrin-Polylysin-Konjugaten: DNA (Transferrin-Polylysin450)
- Fig.2:: Einfluß des Verhältnisses
Transferrin:Polylysin auf die Effizienz des Gentransfers (A: Transferrin-Polylysin200; B: Transferrin-Polylysin450)
- Fig.3:: Bestimmung der erforderlichen Mindest-Anzahl von Transferrin-Molekülen pro DNA-Molekül. A: Ersatz von zunehmenden Anteilen von Konjugat durch nicht-konjugiertes Polylysin. B: Zusatz von nicht-konjugiertem Polylysin zu einer konstanten optimalen Konjugat-Menge
- Fig. 4:: Gentransfer mittels Konjugaten aus Transferrin und synthetischem Protamin
- Fig.5:: Verbesserung der Effizienz der Transferrinfektion mit Konjugaten aus Transferrin und synthetischem Protamin
- Fig.6:: Steigerung der Effizienz der Transferrinfektion in Abhängigkeit vom Zustand der Zellen
- Fig.7:: Einfluß von nicht-kovalent gebundenem Histon H4 auf die Effizienz des Gentransfers
- Fig.8:: Gentransfer in CD4 exprimierende HeLa-Zellen mittels gp120-Polylysin-Konjugaten
- Fig.9:: Steigerung der Effizienz des Gentransfers mittels gp120-Polylysin-Konjugaten durch Zusatz von nicht-kovalent gebundenem Polylysin
- Fig.10:: Steigerung der Effizienz des Gentransfers mittels Transferrin-Polylysin-Konjugaten durch Zusatz von lipophil modifiziertem Polylysin
- Fig.11:: Steigerung der Effizienz des Gentransfers mittels Transferrin-Ethidiumdimer-Konjugaten durch nicht-kovalent gebundenes Polylysin
- Fig.12:: Einfluß von Laktose-modifiziertem Polylysin auf den Gentransfer mittels Transferrin-Polylysin-Konjugaten

Die vorliegende Erfindung wird anhand der folgenden Beispiele illustriert:

### Beispiel 1

### Herstellung von Transferrin-Polylysin200- und Transferrin-Polylysin 450-Konjugaten (TfpL200 und TfpL450)

Die Kopplung erfolgte analog literaturbekannten Methoden (Jung et al., 1981) durch Einführung von Disulfidbrücken nach Modifizierung mit Succinimidylpyridyldithiopropionat. Es wurde vorgegangen, wie in der EP-A1 0388 758 beschrieben, wobei lediglich die Isolierungsmethode geringfügig abgewandelt wurde, wodurch eine höhere Konjugat-Ausbeute erzielt werden konnte.

### a) 3-(2-Pyridyldithio)propionat-modifiziertes Transferrin:

6 ml einer über Sephadex G-25 gelfiltrierten Lösung von 120 mg (1.5 µMol) Transferrin (Humantransferrin, Sigma, eisenfrei) in 3 ml 0.1 M Natriumphosphat-Puffer (pH 7.8) wurden unter starkem Schütteln mit 200 µl einer 15 mM ethanolischen Lösung von Succinimidyl 3-(2-pyridyldithio)propionat (3 µM, SPDP, Pharmacia) versetzt und 1 h bei Raumtemperatur und gelegentlichem Schütteln reagieren gelassen. Über eine Gelsäule (Sephadex G-25, 14 x 180 mm, 0.1 M Natriumphosphat-Puffer pH 7.8 ) wurden niedermolekulare Reaktionsprodukte und Reagensreste abgetrennt und dabei 7 ml der Produktfraktion erhalten; der Gehalt von an Transferrin gebundenem Pyridyldithiopropionatresten wurde anhand eines Aliquots nach Reduktion mit Dithiothreitol durch photometrische Bestimmung der Menge an freigesetztem Pyridin-2-thion ermittelt und betrug ca. 2.6 µMol.

### b) Herstellung von modifiziertem Polylysin200 (pL200) und Polylysin450 (pL450)

Eine gelfiltrierte Lösung von 0.57 µMol pL200 (mit einem durchschnittlichen Polymerisationsgrad von 200 Lysinresten, mit Fluoreszenzmarkierung) in 3 ml 20 mM Natriumacetatpuffer wurde durch Zusatz von 300 µl 1M HEPES Puffer auf pH 7.9 eingestellt. Unter starkem Rühren wurden 204 µl einer 10 mM ethanolischen Lösung von SPDP (2.04 µMol) hinzugefügt. Eine Stunde später wurden 500 µl 1M Natriumacetat pH 5 zugefügt. Zur Abtrennung von niedermolekularen Substanzen wurde über Sephadex G-25 filtriert (Eluens : 20 mM Natriumacetat-Puffer pH 5.0). Es wurde eine Lösung erhalten, die 0.54 µMol pL200 mit 1.86 µMol Dithiopyridingruppen (3.5 Linker pro Polylysinkette) enthielt, mit Puffer auf pH ca. 7 gebracht, 36 mg Dithiothreitol zugegeben und 1 h bei Raumtemperatur unter Argon in der Dunkelheit stehen gelassen. Es wurden 400 µl 3M Natriumacetatpuffer pH 5.0 zugegeben und durch weitere Gelfiltration (Sephadex G-25, 14 x 180 mm Säule, 15 mM Natrium-acetat-Puffer pH 5.0) von überschüssigem Reduktionsmittel abgetrennt. Es wurden 4 ml Produktlösung, enthaltend 0.50 µmol fluoreszenzmarkiertes Polylysin mit einem Gehalt von 1.84 µMol Mercaptogruppen (photometrische Bestimmung mittels Ellman's Reagens, 5.5'-Dithiobis(2-nitrobenzoesäure) erhalten.

In analoger Weise wurden 0.20 µMol pL450 (mit einem durchschnittlichen Polymerisationsgrad von 450 Lysinresten) mit 0.70 µMol SPDP modifiziert, wobei ein Produkt von 0.18 µMol pL450 mit 0.57 µMol Dithiopyridingruppen (3.2 Linker pro Polylysinkette) erhalten wurde.
Die Dithiopyridingruppen wurden mit Dithiothreitol reduziert, wie oben für Polylysin200 angegeben. Es wurden 0.175 µmol Polylysin, modifiziert mit 0.57 µmol Mercaptogruppen, erhalten.

### c) Herstellung von Transferrin-Polylysin-Konjugaten

Transferrin-pL200-Konjugate wurden hergestellt, indem 1.06 µMol des in a) erhaltenen modifizierten Transferrins (in 100 mM HEPES-Puffer pH 7.9) mit 0.20 µMol des in b) erhaltenen modifiziertem pL200 (in 30 mM Natriumacetatpuffer) unter Sauerstoffausschluß in einer Argonatmosphäre gemischt wurden.
In entsprechender Weise wurden Transferrin-Polylysin 450 Konjugate hergestellt, wobei von 0.61 µMol modifiziertem Transferrin gemäß Beispiel 1 a) (in 100 mM HEPES pH 7.9) und 0.12 µMol pL450 (in 30 mM Natriumacetatpuffer) ausgegangen wurde.

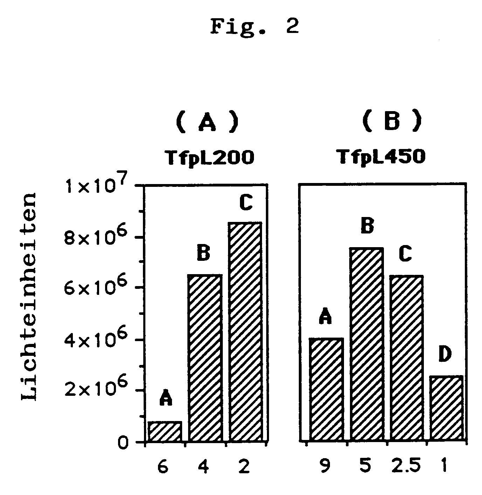

Nach 18 Stunden bei Raumtemperatur wurden die Konjugate durch Kationenaustauschchromatographie (Pharmacia Mono S Säule HR 10/10; Gradientenelution, Puffer A: 50 mM HEPES pH 7.9, Puffer B: Puffer A plus 3M Natriumchlorid) aus der Reaktionsmischung isoliert. Es zeigte sich, daß für die Gewinnung der Polykation-Konjugate wesentlich war, der Reaktionsmischung Natriumchlorid zuzufügen (Endkonzentration 0.6 M im Fall von TfpL200 oder 1M im Fall von TfpL), bevor die Säule beladen wurde, und den Gradienten bei dieser Salzkonzentration zu beginnen. Die Produktfraktionen wurden bei Salzkonzentrationen um ca. 1.4 M im Fall von TfpL200 und ca. 2M im Fall von TfpL450 eluiert. Nach Dialyse gegen HBS (HEPES gepufferte Salzlösung: 20mM HEPES, 150mM NaCl, pH 7.4) wurden die Konjugatfraktionen in einer Gesamtausbeute von 80 % (TfpL200) bzw. 64 % (TfpL450) erhalten.
Die Konjugat-Fraktionen sind hinsichtlich ihres Verhältnisses Transferrin:Polylysin in Fig. 2A und Fig.2B näher charakterisiert.

Der Einbau von Eisen in die Konjugate wurde durchgeführt, indem den Proben 3 - 10 µl 10 mM Eisencitrat[III], 200mM Citrat, mittels Natriumbicarbonat auf pH 7.8 eingestellt) pro mg Transferrinanteil zugefügt wurden.

### Beispiel 2

### a) Bestimmung des optimalen Verhältnisses von Transferrin-Polylysin-Konjugat : DNA

Jedes der im Beispiel 1 erhaltenen Konjugate wurde mittels Titration auf sein im Hinblick auf DNA-Import und -Expression optimales Verhältnis von Konjugat:DNA untersucht. Als DNA wurde pRSVL Plasmid-DNA (De Wet et al., 1987) verwendet, hergestellt mittels Triton-X Lyse Standard-Methode (Maniatis), gefolgt von CsCl/EtBr Gleichgewichtsdichtegradienten-Zentrifugation, Entfärbung mit Butanol-1 und Dialyse gegen 10 mM Tris/HCl pH 7.5, 1 mM EDTA (vgl. EP-A1 0388 758).

Die in den Titrationen verwendeten Transferrin-Polykation/DNA-Komplexe wurden hergestellt, indem eine konstante Menge von pRSVL-Plasmid-DNA, und zwar 10 µg im Falle der TfpL200-Fraktionen und 6 µg im Fall der TfpL450-Fraktionen, mit steigenden Mengen der jeweiligen Konjugat-Fraktionen gemischt wurde.

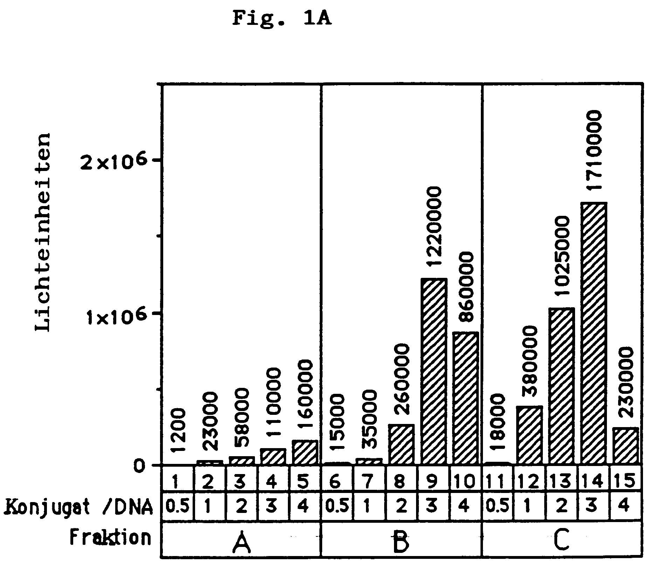

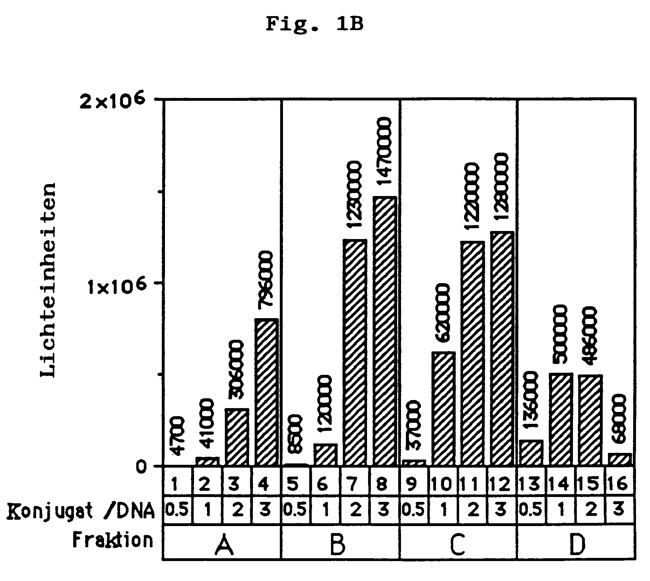

Fig. 1A (TfpL200) und Fig. 1B (TfpL450) zeigen die für die Durchführung der Titrationen gewählten Mengenverhältnisse, wobei die Mengen als Masseverhältnis von DNA zu im Konjugat enthaltenem Transferrin und die Konjugat-Fraktionen mit A - C bzw. A - D (vgl.Fig. 2) bezeichnet sind. Die Komplexe wurden in 500 µl Reaktionsvolumen HBS (150 mM NaCl, 20 mM HEPES pH 7.4) hergestellt, 30 min bei Raumtemperatur inkubiert und zu 2 ml Zellkultur gefügt. Für die Transfektionen wurden Zellen der Erythroleukämie-Zellinie K562 in RPMI 1640 Medium, ergänzt mit 10 % fötalem Kälberserum, 100 IE/ml Penicillin, 100 µg/ml Streptomycin und 2 mM Glutamin, bei einer Dichte von 2 - 5 x 10⁵/Zellen pro ml gezüchtet. Die Zellen wurden 18 - 24 Stunden vor der Transfektion mit 50 µM Desferrioxamin vorbehandelt, um die Zahl der Transferrinrezeptoren auf der Zelle zu erhöhen. Transfektionen wurden mit 5 x 10⁵ Zellen in 2ml Medium in Gegenwart von 100 µM Chloroquin durchgeführt. Nach 4 Stunden Inkubation bei 37°C wurden die Zellen in frisches Medium aufgenommen, bei 37°C inkubiert und für die Luciferase-Bestimmung 18 Stunden später geerntet. In Fig. 1 ist die Luciferase-Aktivität von Zellextrakt-Aliquots (ca. 15 - 20 % des Gesamtextrakts), standardisiert auf Proteingehalt, angegeben.

### b) Einfluß des Transferrin-Polylysin-Verhältnisses in den Konjugaten auf die Effizienz der Transferrinfektion

Drei TfpL200-Konjugat-Fraktionen A - C mit einer unterschiedlichen durchschnittlichen Zahl von Transferrin-Molekülen pro Polylysinkette wurden bei den in a) ermittelten optimierten Mengen (40 µg für die Fraktion A, 30 µg für die Fraktionen B und C) für die Komplexbildung mit 10 µg pRSVL DNA eingesetzt. Mit den erhaltenen Komplexen wurden 10⁵ Zellen unter Standardbedingungen, wie oben angegeben, transfiziert und die Genexpression durch Messung der Luciferase-Aktivität von Zellextrakt-Aliquots, standardisiert auf Proteingehalt, bestimmt.

Transferrinfektions-Versuche mit den vier TfpL450-Fraktionen A - D wurden methodisch genauso durchgeführt, mit dem Unterschied, daß nur 6 µg DNA verwendet wurden und mit den als optimal ermittelten Konjugat-Mengen von 18 µg (Fraktionen A - C) bzw. 12 µg (Fraktion D) gemischt wurden.

Das Ergebnis dieser Versuche ist in Fig. 2 dargestellt (die Werte für die Luciferase-Aktivität beziehen sich auf den Gesamtextrakt). Es zeigt deutlich, daß Fraktionen mit einer niedrigen Anzahl von Transferrin-Molekülen pro Polylysin-Molekül effizienter sind. Fig. 2A: Konjugate C mit einem molaren Verhältnis Transferrin:Polylysin von 2:1 sind 10mal wirksamer als Konjugate A mit einem Verhältnis von 6:1. Fig. 2B: Die Versuche zeigen, daß bei Verwendung des langen Transferrin-pL450-Konjugats Konjugate mit einem Verhältnis von 9:1 weniger aktiv sind als solche mit einem Verhältnis 5:1, wobei diese wiederum in ihrer Aktivität vergleichbar sind mit Konjugaten, die ein Verhältnis Tf:pL von 2.5:1 aufweisen. Bei noch höherem Polylysin-Gehalt (Verhältnis 1:1) hingegen fällt die Aktivität wieder ab.

### Beispiel 3

### Bestimmung der erforderlichen Mindest-Anzahl von Transferrin-Molekülen pro DNA-Molekül

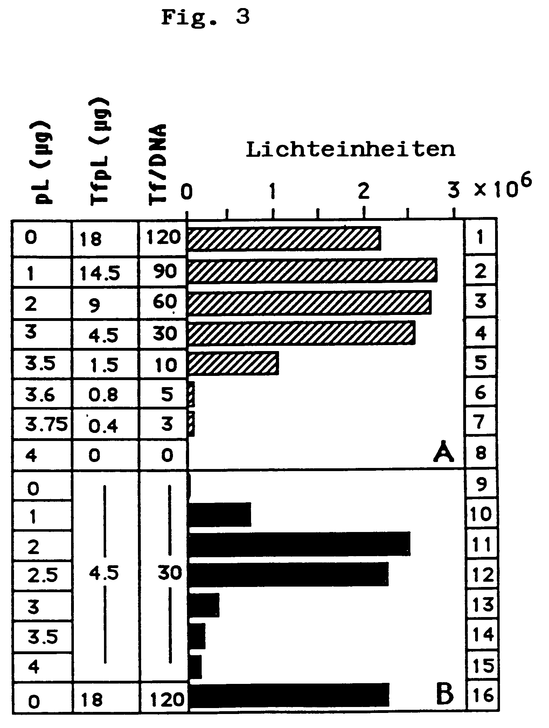

Bei der Durchführung der Versuche im Rahmen dieses Beispiels wurde von der Überlegung ausgegangen, daß die hohe Anzahl von ca. 120 Transferrin-Molekülen pro "doughnut" (diese Anzahl ist ein statistischer Wert, rechnerisch ermittelt aus dem Masseverhältnis Konjugat:DNA) für das Funktionieren der Rezeptor-vermittelten Endozytose nicht erforderlich sein dürfte. Um die Richtigkeit dieser Überlegung zu prüfen, wurden Versuche durchgeführt, in denen ein zunehmender Anteil des Transferrin-Konjugats durch nicht-modifiziertes Polykation ersetzt wurde, wobei die Gesamtpolylysin-Menge beim vorbestimmten Optimum (ca. 4 µg Polylysin pro 6 µg DNA) gehalten wurde. Die Effizienz der Transferrinfektion (Transfektionen und Luciferase-Aktivitätsbestimmungen wurden analog Beispiel 2 durchgeführt) blieb erhalten oder nahm leicht zu, wenn der Konjugat-Gehalt der Probe reduziert und durch Polylysin ersetzt wurde (für Poly(D)lysin und Poly(L)lysin wurden übereinstimmende Ergebnisse erhalten). Wenn jedoch der Transferringehalt unter 10 - 15 Moleküle pro DNA-Molekül sank, nahm auch die Effizienz der Transferrinfektion stark ab (Fig. 3A, Proben 5 - 8). Es wurde ein Minimum von 10 - 15 Transferrinmolekülen pro DNA-Molekül als für effizienten Gen-Transfer erforderlich ermittelt. Wenn der Probe kein Konjugat zugesetzt wird, sinkt der Gen-Transfer praktisch auf Null. Das Ergebnis der Versuche ist in Fig. 3A dargestellt: die Spalten 1 - 8 zeigen die Expression des Luciferase-Gens nach Transferrinfektion, wobei bei der Komplexbildung mit 6 µg DNA die optimale-TfpL200 Menge von 18 µg schrittweise durch steigende Mengen p(D)L240 ersetzt wurde. (Die in Fig. 3 angegebenen Werte für die Luciferase-Aktivität beziehen sich auf den Gesamtextrakt.)

### Beispiel 4

In einer weiteren Serie von Versuchen wurde von einer konstanten Menge (4.5 µg) Transferrin-Polykation-Konjugat ausgegangen, die ca. 1/4 der im Beispiel 2a) als optimal ermittelten Menge entsprach. Das Konjugat wurde mit steigenden Mengen von Polylysin90 (pL90) vermischt und einer konstanten Menge DNA (6 µg) hinzugefügt. Transfektionen und Luciferase-Bestimmung wurden durchgeführt wie in den vorangegangenen Beispielen; das Ergebnis der Versuche ist in Fig. 3B dargestellt. In den Versuchen gemäß Spalten 9 - 15 wurden Komplexe von 6 µg pRSVL mit 4.5 µg TfpL200C hergestellt, denen steigende Mengen von pL90 hinzugefügt wurden. (Spalte 16 zeigt die Ergebnisse des Kontrollversuches mit 6 µg pRSVL und der als optimal ermittelten Menge (18 µg) von TfpL200C.) Wie aus Fig. 3B ersichtlich, konnte die Wirksamkeit der Transfektion, die bei der suboptimalen Ausgangsmenge von TfpL auf ca. 1/100 des optimalen Ergebnisses gesunken war, durch den Zusatz von freiem pL90 wiederhergestellt werden.

### Beispiel 5

Ähnlich wie in Beispiel 4 wurde eine Reihe von Versuchen durchgeführt, wobei von einer suboptimalen Menge von 6 µg DNA, komplexiert mit einer Menge von 9 µg TfpL200 ausgegangen und untersucht wurde, wie verschiedene Mengen unterschiedlicher Polykationen sich auf die DNA-Aufnahme auswirken. Transfektionen und Bestimmung der Effizienz des DNA-Imports wurden unter Standardbedingungen durchgeführt, wie oben angegeben. Als freie Polykationen wurden verschiedene Poly(L)lysine mit einer durchschnittlichen Kettenlänge von 55, 90, 200 oder 450 Lysinmonomeren, Poly(D)lysin mit einer durchschnittlichen Kettenlänge von 240 Lysinen (sämtliche Polylysine wurden in Form von Hydrobromid-Salzen, Sigma, bezogen), Protamin (Lachs-Protamin-Sulfat, histonfrei, Sigma), die Histone H1, H3 und H4 (aus Kalbsthymus, Boehringer Mannheim) eingesetzt. In Tabelle 1 sind die jeweils eingesetzten Mengen der freien Kationen sowie der relative Wirkungsgrad der Transferrinfektion von 6 µg DNA, komplexiert mit einer Mischung von 9 µg TfpL200 plus den angegebenen Mengen von Polykationen, angegeben. Die Zahlen in der Tabelle bedeuten %, bezogen auf den Wirkungsgrad von Komplexen, die mit der optimalen Menge (18 µg) von TfpL200 hergestellt wurden.

Diese Versuchsserie zeigte, daß der Zusatz der Polylysine sowie des natürlichen Protamins und der untersuchten Histone einen zumindest gleichgroßen DNA-Import-Wirkungsgrad erzielte wie die Verwendung der Konjugate, die sich als optimal erwiesen hatten. Ausnahmen bildeten Spermin und Spermidin, die nicht in der Lage waren, das Ausmaß der DNA-Aufnahme bei der physiologischen Salzkonzentration des Gewebekultursystems wiederherzustellen.

### Beispiel 6

### Herstellung von Transferrinkonjugaten mit einem synthetischen Protaminanalogen

Konjugate zwischen Transferrin und einem synthetischen Protamin der Sequenz
Lys-Pro-Arg-Ala-Arg-Arg-Ser-Ser-Ser-Arg-Pro-Val-Arg-Arg-Ser-Ser-Arg-Pro-Ala-Val-Ser-Ala-Arg-Arg-Arg-Ser-Arg-Gly-Gly-Ser-Arg-Arg-Gly-Gly-Gly-Cys
wurden durch Kopplung mittels Disulfidbrückenbildung zwischen dem C-terminalen Cystein des Peptids und 3-(2-Pyridyldithio)propionat-modifiziertem Transferrin synthetisiert.

### a) Herstellung von synthetischem Protamin:

Das Polypeptid wurde durch Festphasensynthese unter Verwendung eines automatischen Peptid-Synthesizers (Applied Biosystems 431A) bei den vom Hersteller empfohlenen Bedingungen synthetisiert (Fluorenmethoxycarbonyl-Technik (Fmoc); als Seitenketten wurden für Serin und Cystein t-Butyl, für Lysin t-Butoxycarbonyl und für Arginin Pentamethylcromansulfonyl eingesetzt). Das erhaltene Rohpeptid wurde einer Gelfiltration (Sephadex G-25 PD 10 Säule, Eluens: 10 % Essigsäure) unterworfen, die erhaltene Lösung mittels Reverse Phase HPLC (Merck-Hitachi, Nucleosil 100-5C18, 8x250 mm. Lösungsmittel A: 0.1 % Trifluoressigsäure in Wasser, Lösungsmittel B: 0.1 % Trifluoressigsäure in 60 % Acetonitril; Gradientenelution mit 0 - 100 % B in 45 min; Durchflußrate 2 ml/min) weiter fraktioniert. Das Produkt eluierte bei einer Konzentration von 43 % Lösungsmittel B und wurde mittels Kationenaustauschchromatographie (Pharmacia MonoS HR 10-10 Säule. Puffer A: 33 mM HEPES pH 7, Puffer B: Puffer A plus 1 M NaCl; Gradientenelution, Durchflußrate 0.5 ml/min) weiter gereinigt. Der Hauptpeak, der bei einer Salzkonzentration von 0.81 M eluierte, wurde durch einen weiteren HPLC-Lauf (gleiche Bedingungen wie oben) entsalzt und lyophilisiert; die Ausbeute betrug 39.6 mg reines Peptid in Form seines Trifluoracetat-Salzes.
Als Flugzeitmassenspektrum (Plasmadesorptionsmassenspektrometrie, PD-MS Instruments, Uppsala, Schweden) wurde ein Wert von 4018.4 (MH⁺, Molekül mit einem t-Butyl-geschützten Cystein) ermittelt.

### b) DNA-Bindungstest

Mittels Gel Mobility Shift Assay wurde bestätigt, daß das in a) hergestellte synthetische Protamin an DNA bindet. Dazu wurden Hind III geschnittene λ-DNA-Fragmente verwendet (Fragmentgrößen zwischen 23120 bp und 564 bp, am 3'-Ende durch Auffüllen der überhängenden Enden mit α³²P dATP durch Klenow-Polymerase während 45 min bei 37°C, Isolierung durch Ethanolfällung nach Zusatz von Glykogen und Natriumacetetat ³²P markiert). Zu jeder Probe mit 2 µl (10 ng) DNA wurden 2.5 µl eines 100 mM HEPES-Puffers pH 7.3, enthaltend 1 M NaCl gefügt und die Proben mit steigenden Mengen (3, 10, 30, 100, 300 und 1000 ng) des synthetischen Peptids (als Trifluoracetat) oder mit 10, 30 oder 100 ng natürlichem Protamin in 10.5 µl wässeriger Lösung gemischt. Nach Zusatz von 2 µl Auftragepuffer (0.25 % Bromphenolblau, 0.25 % Xylencyanol und 30 % Glycerin in Wasser) zu jeder Probe wurden die Proben einer Elektrophorese auf einen 1 % Agarosegel mit 1 x TAE (40 mM Trisacetat/ 1 mM EDTA, pH 8) Laufpuffer bei 50 V (42 mA) unterworfen. Das Gel wurde getrocknet, abschließend wurde eine Autoradiographie während 2 Stunden bei -80°C unter Verwendung eines XAR-Films (Kodak) durchgeführt. Ein deutlicher Band Shift wurde bereits mit 3 ng des synthetischen Peptids, mit 10 ng wurde ein weitestgehender Band Shift (ein Großteil der DNA wurde am Startschlitz zurückgehalten) festgestellt.

### c) Herstellung von Konjugaten zwischen Transferrin und synthetischem Protamin

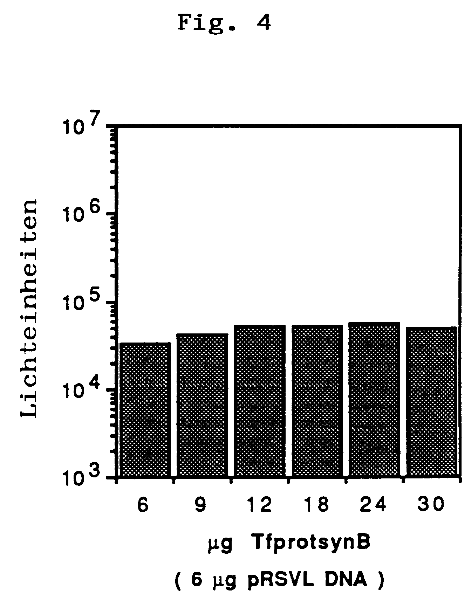

Zu 3 ml einer Lösung, erhalten nach Gelfiltration von 100 mg (1.25 µmol) humanem Transferrin auf einer Sephadex G-25 Säule in 100 mM HEPES pH 7.3, wurden 167 µl einer 15 mM ethanolischen Lösung von Succinimidyl 3-(2-Pyridyldithio)propionat (SPDP; Pharmacia) gegeben und die Lösung gut durchmischt. Nach 1 h bei Raumtemparatur wurde eine weitere Gelfiltration (Sephadex G-25 Säule) mit 20 mM HEPES pH 7.3 durchgeführt, die 4.6 ml einer Lösung von 1.14 µmol Transferrin, modifiziert mit 2.24 µmol Dithiopyridin-Linker, lieferte. Nach Sauerstoffausschluß wurde diese Lösung mit 1.5 ml einer Lösung von 0.9 µmol synthetischen Protamin in freier Mercapto-Form in 0.5 % Essigsäure gemischt. Die Reaktionsmischung wurde durch Kationenaustauschchromatographie (Pharmacia Mono S-Säule HR10/10; Gradientenelution 0.5 ml/min, Puffer A: 50 mM HEPES pH 7.3 Puffer B: Puffer A plus 3 M NaCl, Messung durch Bestimmung der UV-Absorption bei 280 nm) fraktioniert. Der Überschuß von nicht-gekoppeltem Transferrin eluierte zuerst; die Produktfraktionen eluierten während des Gradienten und wurden in zwei Konjugatfraktionen gepoolt: Tf-protsyn A (eluierte zwischen 17 % und 24 % Puffer B) und Tf-protsyn B (eluierte zwischen 24 % und 30 % Puffer B). Diese lieferten nach Dialyse gegen 25 mM HEPES pH 7.3 Tf-protsyn A mit einem Gehalt von 9 mg modifiziertem Transferrin (molares Verhältnis Transferrin:Protamin ca.1:1.3, aufgrund der Aminosäureanalyse nach Proteinhydrolyse) und Tf-protsyn B mit einem Gehalt an modifiziertem Transferrin von 13.5 mg (molares Verhältnis Transferrin:Protamin ca. 1:1.8). Die Konjugate wurden in einer Gesamtausbeute von 0.28 µmol Transferrin erhalten.
Die erhaltenen Konjugate wurden mit pRSVL DNA, wie im Beispiel 2 beschrieben, komplexiert; die Mengenverhältnisse DNA:Konjugat sind aus Fig. 4 ersichtlich. Mit den erhaltenen Komplexen wurden Transfektionen durchgeführt, wie in den vorigen Beispielen beschrieben. Wie Fig.4 zeigt, haben die Protamin-Konjugate eine signifikante, wenn auch im Verhältnis zu den Transferrin-Polylysin-Konjugaten relativ geringe Fähigkeit, DNA in die Zelle zu transportieren. In Fig. 4 ist die Luciferase-Aktivität von Zellextrakt-Aliquots (ca. 15 - 20 % des Gesamtextrakts), standardisiert auf Proteingehalt, angegeben.

### Beispiel 7

### Verbesserung der Effizienz der Transferrinfektion mit Konjugaten aus Transferrin und synthetisches Protamin durch teilweisen Ersatz mit Polylysin

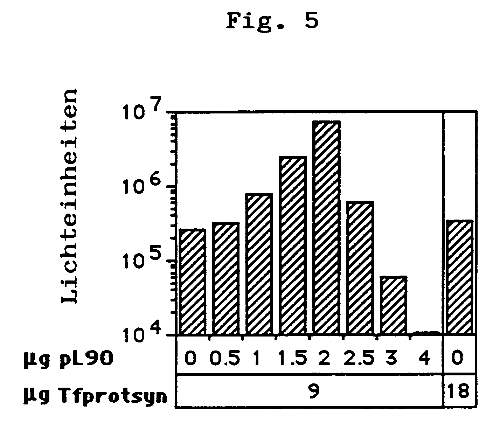

Die mit 6 µg pRSVL DNA gebildeten Komplexe (die Konjugat/DNA-Verhältnisse sind aus Fig.4 ersichtlich) wurden mit pL90 vermischt und unter denselben Bedingungen wie in den vorangegangenen Beispielen verwendet, um DNA in K562-Zellen (ATCC Nr. CCL 243) zu transportieren. Das Ergebnis der durchgeführten Experimente ist aus Fig. 5 ersichtlich; die Werte für die Luciferase-Aktivität beziehen sich auf den Gesamtextrakt: wenn Teile der synthetisches Protamin enthaltenden Konjugate durch freies Polylysin ersetzt werden, kann eine Zunahme der DNA-Expression um bis zum ca. 20-fachen beobachtet werden. (Zum Vergleich ist das Experiment mit der vorher ermittelten optimalen Menge gezeigt; rechte Spalte in Fig. 5).

### Beispiel 8

### Steigerung der Effizienz der Transferrinfektion in Abhängigkeit vom Zustand der Zellen

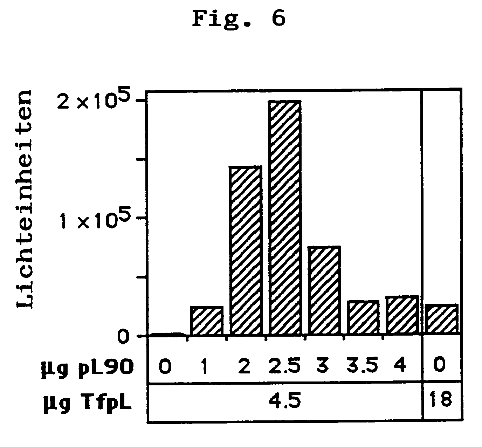

Im Rahmen dieses Beispiels wurde untersucht, inwieweit das Ausmaß der Verbesserung der Transferrinfektion durch "Verdünnung" mit Polylysin vom Zustand der Zellen abhängt. Die Versuche entsprachen grundsätzlich denen in Beispiel 4: ausgehend von 4.5 µg TfpL200C, wurden die in die Zelle zu transportierenden Komplexe unter Zusatz steigender Mengen pL90 gebildet, wobei im Unterschied zu Beispiel 4 die K562-Zellen nicht mit Desferrioxamin vorbehandelt wurden. Die DNA-Menge in den Komplexen betrug in allen Proben 6 µg. Die für diese Versuche verwendeten Zellen wurden auf ihre Transferrinrezeptorzahl untersucht (Scatchard Analyse); sie betrug ungefähr 1/5 der von Desferrioxamin-behandelten Zellen. Wie mittels optimierten Standardbedingungen (18 µg TfpL200 für 6 µg pRSVL DNA) festgestellt worden war, ist als Folge der verminderten Rezeptorzahl eine Verringerung der DNA-Expression feststellbar. Wenn jedoch im Falle der nicht mit Desferrioxamin stimulierten Zellen 3/4 des Transferrin-Polylysin-Konjugats durch Polylysin ersetzt wird, nimmt die DNA Expression um mehr als das 8-fache zu. Dies entspricht einem Wert, der nur um das 1,8-fache geringer ist als bei Zellen mit aufgrund Desferrioxamin-Stimulierung erhöhter Rezeptorzahl. Das Ergebnis dieser Versuche ist in Fig. 6 dargestellt. Bei Desferrioxamin-stimulierten Zellen wird die Transferrin-vermittelte DNA-Aufnahme durch Zugabe von freiem Polylysin90 nicht wesentlich verbessert.

### Beispiel 9

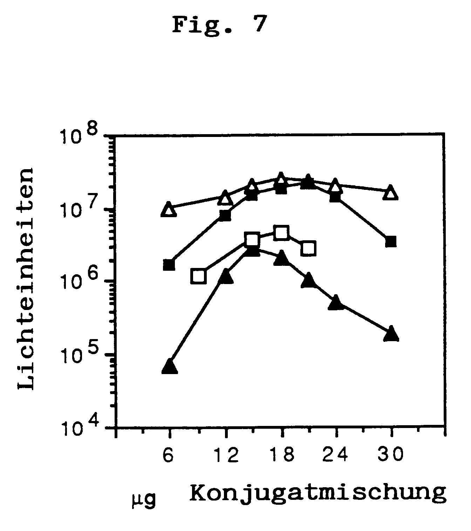

Um den in Beispiel 4 beobachteten Effekt der durch freies Histon H4 bewirkten Zunahme der Genaktivität um das ca. 5-fache (Tabelle 1) näher zu untersuchen, wurde der folgende Versuch durchgeführt: es wurden Stammischungen von TfpL200C-Konjugaten mit 1 (offene Dreiecke), 2 (geschlossene Quadrate) oder 4 (geschlossene Dreiecke) Masseäquivalenten Histon H4 hergestellt und in einer Art Titration für jede einzelne Mischung die für Komplexbildung mit einer konstanten DNA-Menge (6 µg) jeweils optimalen Mengen bestimmt (Fig. 7). Die optimale Genaktivität, bewirkt durch eine 4/1 Mischung von Histon H4/TfpL ist vergleichbar mit der Aktivität, die durch das Konjugat allein (offene Quadrate) erzielt wird. Die elektronenmikroskopische Untersuchung dieser Komplexe zeigte eine nur schwache Kondensation der DNA. Elektronenmikroskopie der H4/TfpL 1/1-Komplexe zeigte eine zwar stärkere, jedoch immer noch wesentlich geringere Kondensation als die reinen Konjugat-DNA-Komplexe. Überraschenderweise betrug jedoch die Steigerung der Transferrinfektion bei Verwendung der 1/1 H4/TfpL Mischung das ca. 7-fache (Transfektionen wurden unter Standardbedingungen wie in Beispiel 2 durchgeführt; die Werte für die Luciferase-Aktivität beziehen sich auf den Gesamtextrakt).

### Beispiel 10

### Transport von DNA in CD4⁺-Zellen

### a) Herstellung von gp120-Polylysin 190-Konjugaten

Die Kopplung erfolgte (entsprechend der Herstellung der Transferrin-Konjugate) analog literaturbekannten Methoden entweder durch Einführung von Disulfidbrücken nach Modifizierung mit Succinimidyl-Pyridyldithiopropionat oder durch Thioether-Verknüpfung nach Modifizierung mit 6-Maleimidocapronsäure-N-hydroxysuccinimidester (EMCS, Sigma) (Fujiwara et al., 1981).

Disulfidverknüpfte gp120-Polylysin 190-Konjugate:
Eine Lösung von 3 mg rekombinantem gp120 (hergestellt nach der von Lasky et al., 1986, beschriebenen Methode) in 50 mM HEPES pH 7.8 wurde mit 7 µl 10 mM ethanolischer Lösung von SPDP versetzt. Nach 1 h bei Raumtemperatur wurde über eine Gelsäule Sephadex G 25 filtriert (Eluens 100 mM HEPES-Puffer pH 7.9) und man erhielt dabei 2.8 mg (23 nmol) rgp120, modifiziert mit 67 nmol Pyridyldithiopropionatresten.
Eine Lösung von 6.6 nmol Polylysin 190, fluoreszenzmarkiert und modifiziert mit 23 nmol Mercaptogruppen (nach Reaktion mit SPDP, Behandlung mit Dithiothreitol und nachfolgende Gelfiltration), in 120 µl 30 mM Natriumacetat wurde unter Sauerstoffausschluß mit dem modifizierten rgp120 gemischt und über Nacht bei Raumtemperatur stehen gelassen. Das Reaktionsgemisch wurde durch Zugabe von 5M NaCl auf einen Gehalt von ungefähr 0.6 M gebracht. Die Isolierung der Konjugate erfolgte durch Ionenaustauschchromatographie (Mono S, Pharmacia, 50mM HEPES pH 7.3, Salzgradient 0.6 M bis 3 M NaCl); nach Fraktionierung und Dialyse gegen 25 mM HEPES pH 7.3 wurden zwei Konjugatfraktionen A und B, bestehend aus 0.33 mg rgp120 modifiziert mit 1.3 nmol Polylysin 190 (im Falle der Fraktion A), bzw. 0.34 mg rgp120 modifiziert mit 3.2 nmol Polylysin 190 (Fraktion B) erhalten.

### Thioether-verknüpfte gp120-Polylysin 190-Konjugate:

Eine Lösung von 2 mg rekombinantem gp120 in 0.45 ml 100 mM HEPES pH 7.9 wurde mit 17 µl einer 10 mM Lösung von EMCS in Dimethylformamid versetzt. Nach 1 h bei Raumtemperatur wurde über eine Gelsäule Sephadex G 25 filtriert (Eluens 100 mM HEPES-Puffer 7.9). Die Produktlösung (1.2 ml) wurde sogleich unter Sauerstoffausschluß umgesetzt mit einer Lösung von 9.3 nmol Polylysin 190, fluoreszenzmarkiert und modifiziert mit 30 nmol Mercaptogruppen (in 90 µl 30 mM Natriumacetat pH 5.0), und über Nacht bei Raumtemperatur stehen gelassen. Das Reaktionsgemisch wurde durch Zugabe von 5M NaCl auf einen Gehalt von ca. 0.6 M gebracht. Die Isolierung der Konjugate erfolgte durch Ionenaustauschchromatographie (Mono S, Pharmacia, 50mM HEPES pH 7.3, Salzgradient 0.6 M bis 3 M NaCl); nach Fraktionierung und Dialyse gegen 25 mM HEPES pH 7.3 wurden drei Konjugatfraktionen A, B und C erhalten, bestehend aus 0.40 mg rgp120, modifiziert mit 1.9 nmol Polylysin 190 (im Falle der Fraktion A), bzw. 0.25 mg rgp 120, modifiziert mit 2.5 nmol Polylysin 190 (Fraktion B), bzw. 0.1 mg rgp 120, modifiziert mit 1.6 nmol Polylysin 190 (Fraktion C).

### b) Herstellung von gp120-Polylysin/DNA-Komplexen

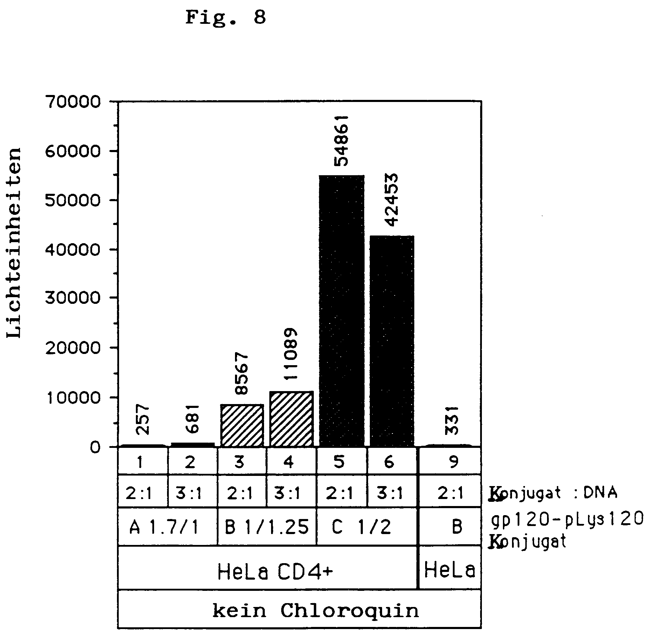

Die Komplexe wurden hergestellt, indem zunächst 6 µg DNA in 330 µl HBS bei Raumtemperatur verdünnt wurden (100 µg/ml oder weniger). Als DNA wurde pRSVL Plasmid-DNA verwendet (vgl. Beispiel 2). Aliquots der gp120-pL190 Konjugate (die Mengen sind in Fig. 8 angegeben) wurden in 170 µl HBS verdünnt. Die jeweilige Konjugatverdünnung wurde rasch der DNA-Verdünnung hinzugefügt, 30 min inkubieren gelassen und anschließend für die Transfektion verwendet.

### c) Transfektion von CD4⁺ Zellen

CD4⁺ HeLa-Zellen (ATCC Nr. CCL2; Maddon et al., 1986) wurden zu 6x10⁵ Zellen pro T-25-Flasche in DEM-Medium plus 10 % FCS (fötales Kälberserum) ausgesät.
18 h später wurden die Zellen zweimal mit DEM-Medium ohne Serum gewaschen und in diesem Medium (5 ml) 5 h lang bei 37°C bebrütet. Daraufhin wurden die Lösungen der gp120-pL/pRSVL Komplexe den Zellen beigegeben. Nach 4 Stunden wurde ein gleiches Volumen DME-Medium (Dulbecco's modified Eagle's Medium) mit einem Gehalt von 10 % fötalem Kälberserum zu jeder Probe gegeben. Nach 24 Stunden wurden die Zellen geerntet, Extrakte hergestellt und Aliquots entsprechend gleichem Proteingehalt (ca. 1/5 des Gesamtmaterials) wie in den vorigen Beispielen auf Luciferaseaktivität untersucht. Die in Fig. 8 angegebenen Werte entsprechen der Luciferaseaktivität von 6 x 10⁵ Zellen. Es wurde festgestellt, daß die Aktivität der gp120-pL Konjugate vom Verhältnis der Komponenten abhängt, wobei größere Aktivität bei den Konjugaten mit niedrigem gp120 : Polylysin-Verhältnis (Fraktion C, Spuren 5 und 6) und eine sehr geringe oder keine Aktivität bei der Fraktion mit einem hohen gp120 : Polylysin-Verhältnis (Fraktion A, Spuren 1 und 2) festgestellt wurde.

### Beispiel 11

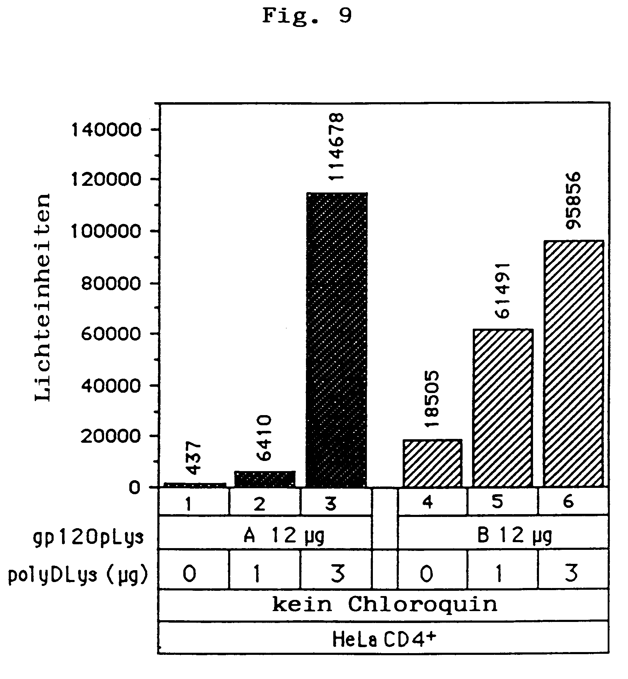

Diejenigen gp120-pL Konjugate, die im Beispiel 10 schlechte Ergebnisse bei der Transfektion gezeigt hatten (Fraktionen A und B) wurden daraufhin untersucht, ob ein Zusatz von freiem Polylysin die DNA-Aufnahme verbessern kann. Es wurden 6 µg DNA und 12 µg Konjugat verwendet, wobei dem Konjugat jeweils vor der Komplexierung mit DNA 1 oder 3 µg Polylysin240 zugesetzt wurden. Übereinstimmend mit den für die Transferrin-Konjugate erhaltenen Ergebnissen wurde eine starke Zunahme der Luciferase-Aktivität (260-fach oder 5,2-fach) beobachtet (Fig. 9).

### Beispiel 12

### A) Synthese von N^{ε}-Undecyl-Polylysin (pL200-C11)

Das Trifluoracetatsalz von Polylysin mit einer durchschnittlichen Kettenlänge von 200 Lysinmonomeren pL200 wurde hergestellt, indem 42 mg pL200 (Hydrobromidsalz, Sigma) in 1 ml 0.5 % wässeriger Trifluoressigsäure gelöst, 0.2 ml 0.3 M wässrigem N-Ethyldiisopropylamin/TFA Puffer (pH 8) zugefügt wurde und mit 0.5 % wässeriger Trifluoressigsäure über eine Sephadex G25 Säule (170 x 14 mm) filtriert wurde. Die gesammelte Produktfraktion (Ninhydrinassay) wurde lyophilisiert und ergab 44 mg (91 %) pL200 (TFA-Salz). Zu einer Lösung von 22 mg (0.48 µmol) dieses Salzes in 3.5 ml Dioxan/Wasser (4/1) wurde bei Raumtemperatur eine Lösung von 4.1 mg (24 µmol) Undecanal (Sigma) in 50 µl Dioxan (Merck, z.A. Qualität, vor Verwendung frisch destilliert). Anschließend wurden zwei Portionen zu 12 mg Natriumcyanoborhydrid in einem Abstand von 4 h zugefügt. Die Reaktionsmischung wurde bei Raumtemperatur 5 Tage stehen gelassen. Anschließend wurden 200 µl Essigsäure zugefügt und das Lösungsmittel im Vakuum abgedampft. Der Rückstand wurde in 1.2 ml Wasser/Essigsäure/Ethanol (65/10/25) aufgenommen und mit Wasser/Essigsäure/Ethanol (77/3/20) über Sephadex gelfiltriert (G25-PD10). Die Produktfraktionen (Ninhydrinassay) wurden lyophilisiert und ergaben 19.7 mg pL200 (als Acetat), modifiziert mit ca. 50-80 Undecylgruppen pro Molekül (berechnet aus der Ninhydrinanalyse und NMR-Analyse). Die NMR-Analyse (¹H-NMR ;D₂0 + 15 % Tetradeuteromethanol) ergab folgende Werte: δ (ppm)= 4.15 - 4.4 (Lysin α-H), 3.28 (von Deuteromethanol), 2.9 - 3.1 (Lysin ε-H und H (C-1, Undecyl)), 1.96 (Acetat-CH₃),
1.1 - 2.0 (aliphatisches CH₂ von Lysin und Undecyl), 0.7 - 0.9 (Undecyl).

### B) Zellkultur und Transfektion

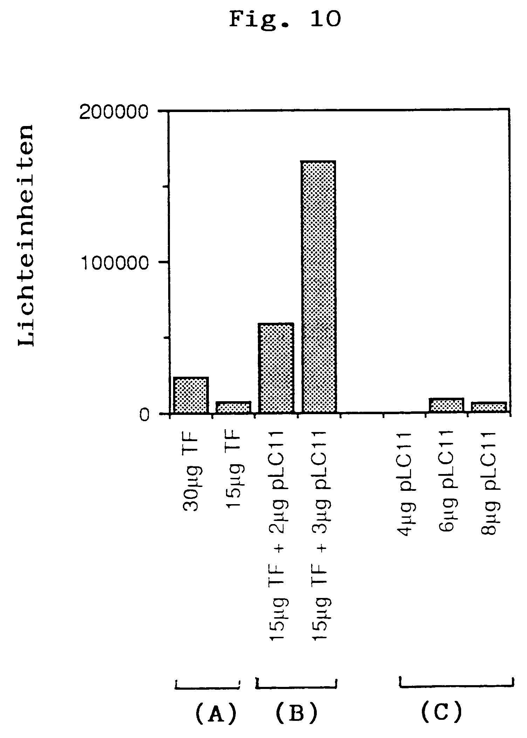

Transfektionen wurden mit 500.000 HepG2-Zellen (ATCC Nr. HB 8065; Knowles et al., 1980) in 5 ml DMEM plus 10 % fötalem Kälberserum in Gegenwart von 100 µM Chloroquin durchgeführt. (Die Zellen wurden erhalten, indem sie trypsinisiert, mit 10 ml DMEM Medium plus 10 % fötalem Kälberserum in Flaschen ausgesät und 24 h lang bei 37°C wachsen gelassen wurden. Dann wurde das Medium durch 10 ml frisches Medium ersetzt, die Zellen weitere 24 h bei 37°C wachsen gelassen und das Medium mit 5 ml frischem Medium ersetzt).
a) Es wurden DNA/Konjugat-Komplexe gebildet, indem 330 µl einer Lösung von 10 µg pRSVL in HBS mit 170 µl einer Lösung von 15 µg bzw. 30 µg (optimale Menge) von TfpL200 Konjugaten in HBS gemischt wurden. Die erhaltenen Komplexe wurden für den Gentransfer in die Zelle verwendet, wie in den vorangegangenen Beispielen beschrieben. Nach 4-stündiger Inkubation bei 37°C wurden die Zellen gewaschen und in frisches Medium überführt, bei 37°C inkubiert und 18 h später für die Luciferasebestimmung geerntet. Aliquots der Zellextrakte, standardisiert auf Proteingehalt, wurden auf ihre Luciferaseaktivität untersucht; die Werte, berechnet für den Gesamtextrakt, sind in Fig. 10A wiedergegeben.
b) Es wurden DNA-Konjugat-Komplexe mit einem Gehalt an nicht kovalent gebundenen, modifizierten Polykationen hergestellt, indem 330 µl einer Lösung von 10 µg pRSVL in HBS mit 170 µl einer Lösung, enthaltend eine Mischung von 15 µg TfpL200-Konjugaten mit verschiedenen Mengen pL200-C11 in HBS gemischt wurden. Die Komplexe wurden unter den oben beschriebenen Bedingungen für den Gentransfer verwendet; die Berechnung der Luciferasewerte erfolgte ebenfalls wie oben und ist in Fig. 10B wiedergegeben (Fig. 10C zeigt als Vergleich die Werte, erhalten mit pL200-C11 allein).

### Beispiel 13

### A) Herstellung von Transferrin-Ethidiumdimer-Konjugaten (Tf-EtD)

Eine Lösung von 32 mg (0.4 µmol) Transferrin (human, eisenfrei, Sigma) in 1 ml 100 mM Natriumacetat-Puffer pH 5 wurde auf einer Sephadex G-25 Säule gelfiltriert. Die erhaltenen 1.9 ml Lösung wurden auf 0°C gekühlt, worauf 80 µl 30 mM Natriumacetat-Puffer pH 5, enthaltend 1.3 mg (6 µmol) Natriumperjodat, hinzugefügt wurden. Die Mischung wurde in einem Eisbad bei Dunkelheit 90 min stehen gelassen. Zur Entfernung der niedermolekularen Produkte wurde eine weitere Gelfiltration (Sephadex G-25, 30 mM Natriumacetat-Puffer pH 5) durchgeführt, die eine Lösung mit einem Gehalt von ca. 20 mg (0.25 µmol) oxidiertes Transferrin, ergab (UV-Absorption bei 280 nm und Ninhydrinassay; die oxidierte Form, die Aldehyde enthält, ergibt im Unterschied zum nicht modifizierten Transferrin eine Farbreaktion mit Anisaldehyd-Reagenz: die Probe wird auf eine Kieselgel-Dünnschichtplatte getropft, getrocknet in p-Anisaldehyd/Schwefelsäure/Ethanol 1:1:18 getaucht, getrocknet, und erhitzt). Die Transferrinlösung wurde einer Lösung, enthaltend 1 mg (1.17 µmol) Ethidium-Homodimer (5.5'-Diazadecamethylen-bis (3.8-diamino-6-phenyl-phenanthridium)dichlorid, di HCl, E-1169, Molecular Probes) in 1.2 ml Wasser hinzugefügt. Die Mischung wurde in der Dunkelheit gehalten, der pH-Wert der Lösung auf 7.3 eingestellt (Zusatz von HEPES-Puffer). Daraufhin wurden 2 Portionen von 1 mg (16 µmol) Natriumcyanoborhydrid in je 50 µl Wasser in 4-stündigem Abstand hinzugefügt. Die Reaktionsmischung wurde bei Raumtemparatur und Dunkelheit 2 Tage 3 Nächte stehen gelassen und anschließend gelfiltriert (Sephadex G-25 50 mM HEPES pH 7.3). Mittels UV-Absorptionsmessung bei 495 nm wurde festgestellt, daß in diesem Schritt ca. 200 µg nicht-konjugiertes Ethidiumdimer zusammen mit anderen niedermolekularen Substanzen entfernt wurden.
Ein Drittel der 4.1 ml Lösung, die das Konjugat enthielt, wurde für Reinigungsexperimente verwendet. Die verbleibenden 2/3 wurden mit 0.6 M Guanidinhydrochlorid (mit Natriumacetat auf pH 5 gepuffert) verdünnt und auf eine Säule, die auf Basis hydrophober Wechselwirkung trennt, aufgetragen (Phenyl-Sepharose CL-4B Pharmacia, 100 x 8 mm, ca. 4.5 ml). Eine schwach rosafarbene Lösung eluierte im Durchfluß, der auch 1.5 mg Transferrin enthielt. Die violett gefärbten Konjugate blieben auf der Säule und wurden (nach Waschen mit 50 ml 0.6 M Guanidinhydrochlorid) mit 0.6 M Guanidinhydrochlorid, enthaltend zuerst 2 % und anschließend 3 % Octylglucopyranosid (Sigma) eluiert. Die Konjugate wurden als zwei nacheinander eluierte Fraktionen (7 ml und 27.5 ml) gesammelt, 3 Tage lang gegen 2 l 200 mM Guanidinhydrochlorid dialysiert. Daraufhin wurde auf dem Speedvac (Savant) auf ein Volumen von je 3 ml eingeengt und weitere zwei Male 2 Tage lang dialysiert (2 l 25 mM HEPES pH 7.3). Diese Vorgangsweise lieferte (trotz Verlusten aufgrund der ausgedehnten Dialyse) zwei Transferrin-Ethidiumdimer-Konjugatfraktionen, enthaltend je 2 mg Tansferrin (Bestimmung sowohl durch Ninhydrinassay als auch durch Bio-Rad Protein Assay); die beiden violett gefärbten Fraktionen hatten 0.42 AU (522 nm) bzw. 0.68 AU (516 nm). (Da das Transferrin-konjugierte Ethidiumdimer seine UV-Absorptionseigenschaften geändert hatte, konnte der genaue Gehalt von Ethidiumdimer in den Konjugaten nicht bestimmt werden).
Der Einbau von Eisen wurde analog Beispiel 1 durchgeführt.

### B) Zellkultur und Transfektion

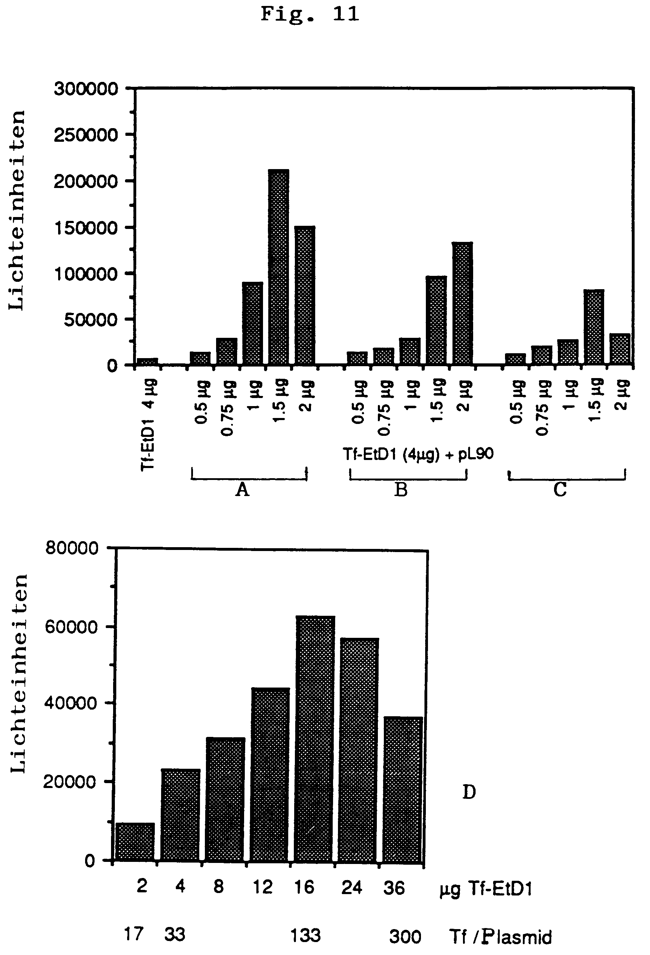

a) DNA/Konjugat-Komplexe wurden hergestellt, wie für TfpL-Konjugate beschrieben, indem 330 µl Lösung von 6 µg pRSVL in HBS mit 170 µl Lösung verschiedener Menge der Tf-EtD-Konjugate (Konjugatfraktion 1) in HBS gemischt wurden (Interkalierung) und anschließend 170 µl einer Lösung verschiedener Mengen pL90 (Kondensation) hinzugefügt wurden. Die erhaltenen Komplexe wurden für die Transfektion von 500.000 K562 Zellen unter Standardbedingungen verwendet; Aliquots von Zellextrakten, standardisiert nach Proteingehalt, wurden auf Luciferaseaktivität untersucht ( Fig. 11A). (Fig. 11D zeigt das Ergebnis, das mit Tf-EtD-Konjugaten allein erzielt wurde).
b) Komplexe mit einem Gehalt an nicht kovalent gebundenen Polykationen wurden gebildet, indem zuerst 170 µl einer Lösung von 4 µg Tf-EtD-Konjugaten mit 170 µl einer Lösung verschiedener Mengen pL90 in HBS gemischt und anschließend eine Lösung von 170 µl einer Lösung von 6 µg pRSVL in HBS (Interkalierung plus Kondensation) zugefügt wurden. Transfektion und Luciferasebestimmung wurden wie oben beschrieben durchgeführt (Fig. 11B).
c) Komplexe mit einem Gehalt an nicht kovalent gebundenen Polykationen wurden gebildet, indem zuerst 170 µl einer Lösung von 6 µg pRSVL in HBS mit 170 µl einer Lösung verschiedener Mengen pL90 (Kondensation) gemischt und anschließend 170 µl einer Lösung von 4 µg Tf-EtD-Konjugaten (Interkalierung) zugefügt wurden. Transfektion und Luciferasebestimmung wurden wie oben beschrieben durchgeführt (Fig. 11C).

### Beispiel 14

### a) Herstellung von N^{ε}-lactosyliertem Polylysin (pL-Lactose)

Zu einer Lösung von 16.5 mg (0.44 µmol) Poly(L)lysin mit einer durchschnittlichen Kettenlänge von 200 Lysinmonomeren (pL200), Acetatsalz, in 0.46 ml 100 mM Natriumacetat (pH 5.0) wurden 90 mg Lactose (Sigma) hinzugefügt, die sich während 30 minütigem Rühren bei 37°C lösten. Während die Lösung bei dieser Temperatur gehalten wurde, wurden vier Portionen von je 3 mg Natriumcyanoborhydrid in Abständen von ca. 10 h hinzugefügt. Die Reaktionsmischung wurde während weiterer 21 h bei 37°C stehengelassen. Dann wurden 15 µl Essigsäure hinzugefügt und die Reaktionsmischung einer Gelfiltration (Sephadex G25-PD10) mit 100 mM Natriumacetat (pH 5.0) unterworfen. Die Produktfraktionen (Nachweis mit Ninhydrintest und Färbung mit Anisaldehyd) wurden über Nacht gegen 20 mM Natriumacetat und anschließend gegen 0.05 % Essigsäure dialysiert. Abschließende Lyophilisierung ergab 26.5 mg pL200, Acetatsalz, mit ca. 70 % der N^{ε}-Lysin-Aminogruppen lactosyliert, wie mit Hilfe der Ninhydrinanalyse und NMR-Analyse geschätzt wurde.

¹H-NMR (D20, 250 MHz, Unterdrückung des Lösungsmittelsignals):
δ (ppm): 4.51 (d, J=7.2 Hz; Galaktose-1H), 4.22 - 4.34 (Lysin α-H), 4.1 - 4.22 (Zucker H), 2,9 - 4.0 (Lysin ε-H und Zucker H), 1.91 (Acetat-CH₃), 1.1 - 2.0 (aliphatische Lysin-CH₂).

### b) Zellkultur und Transfektion:

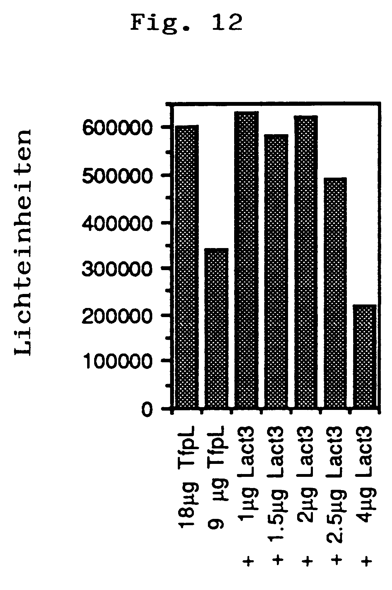

Die Transferrinfektion wurde mit 500 000 K562 Zellen und 6 µg pRSVL DNA sowie mit 18 µg TfpL, mit 9 µg TfpL und mit 9 µg TfpL plus Zusatz verschiedener Mengen pL-Lactose nach Standardbedingungen wie in den vorangegangenen Beispielen durchgeführt, die mit dem Luciferase-Assay erhaltenen Aktivitäten sind in Fig.12 wiedergegeben (die Angabe der Licht-Einheiten bezieht sich auf Zellextrakt-Aliquots (ca. 15 - 20 % des Gesamtextrakts), standardisiert auf Proteingehalt). Es zeigt sich, daß der Zusatz von nicht-kovalent gebundenem Lactose-modifiziertem Polylysin in der Lage ist, die durch den Einsatz einer geringeren Menge Konjugat erzielte Verringerung der Transferrinfektionseffizienz auszugleichen.

### Beispiel 15

### a) Herstellung von N^{ε}-maltosyliertem Polylysin (pL-Maltose)

Es wurde genauso vorgegangen, wie im Beispiel 14 beschrieben mit dem Unterschied, daß Maltose zur Modifizierung von Polylysin eingesetzt wurde. Es wurden folgende ¹H-NMR-Daten erhalten:
δ (ppm): 5.11 (d, J=3.4Hz; Glukose-1H), 2.95-4.4 (Lysin-H und Zucker-H), 1.91 (Acetat CH₃), 1.1-2.0 (aliphatische CH₂ von Lysin).

### b) Einfluß von pL-Maltose auf den Gentransfer

Transferrinfektion von 500.000 K562-Zellen mit 6 µg pRSVL-DNA, durchgeführt wie in Beispiel 14 b) beschrieben, ergab im Fall von komplett mit TfpL gesättigten DNA-Komplexen (12 µg TfpL190B) 1.01x10⁶ Lichteinheiten an spezifischer Luciferaseaktivität (ca. 20 % des Gesamtprobenextraktes), mit halbgesättigten DNA-Komplexen (6 µg TfpL190B) 549.000 Lichteinheiten. Durch Zusatz von 2.5 µg pL-Maltose (6 µg TfpL190B + 2.5 µg pL-Maltose) wurde die Effizienz der Transfektion auf 1,215.000 Lichteinheiten angehoben.

### Beispiel 16

### a) Herstellung von N^{ε}-Zellobiose-modifiziertem Polylysin (pL-Zellobiose)

Es wurde genauso vorgegangen, wie im Beispiel 14 beschrieben mit dem Unterschied, daß Zellobiose zur Modifizierung von Polylysin eingesetzt wurde.

### b) Einfluß von pL-Zellobiose auf den Gentransfer

Bei Transfektion von K562-Zellen unter denselben Bedingungen wie in Beispiel 15b) wurden durch Zusatz von 2 µg pL-Zellobiose (6 µg TfpL190B + 2 µ9 pL-Zellobiose) 1,160.000 Lichteinheiten erhalten.

### Literatur:

Böttger, M. et al., (1988), Biochem. Biophys. Acta 950, 221-228
Chattoraj, D.K. et al., (1978), J.Mol.Biol. 121, 327-337
Ciliberto, G. et al., (1985), Cell 41, 531-540
Darnell, J. et al., (1989) in Molecular Cell Biology, page 567, Second edition, edited by W.H. Freeman and Company, New York
De Wet et al., (1987), Mol.Cell.Biol. 7, 725-737
Farber, F.E. et al., (1975), Biochim.Biophys.Acta 390, 298-311
Fujiwara et al., (1981), J.Immunol.Meth. 45, 195
Ham, R.G., (1965), Proc.Natl.Sci. USA 53, 288
Jung et al., (1981), Biochem.Res.Commun. 101, 599
Knowles, B.B. et al., (1980), Science 209, 497-499
Kurachi, K. und Davie, E.W. (1982), Proc.Natl.Acad.Sci.USA 79, 6461-6464
Laemmli, U.K., (1975), Proc.Nat.Acad.Sci. USA 72, 4288-4292
Lasky et al., (1986), Science 233, 209-212
Lasky et al., (1987), Cell 50, 975-985
Maddon et al., (1986), Cell 47, 333-348
Maniatis et al., (1982), Molecular Cloning, Cold Spring Harbor
Nygren A. et al., (1988), Proc.Natl.Acad.Sci.USA 85, 6543-6546
Pelchen-Mattews et al., (1989), EMBO J. 8, 3641-3649
Riordan, J.R. et al., (1989), Science 245, 1066-1073
Tikchonenko T.I. et al., (1988), Gene 63, 321-330
Valerio, D. et al., (1984), Gene 31, 147-153
Wagner, E. et al., (1991), Bioconjugate Chemistry 2, 226-231
Wu, G.Y. und Wu, C.H., (1987), J.Biol.Chem. 262, 4429-4432
Zamecnik et al., (1986), Proc.Natl.Acad.Sci. 83, 4143
Zon, G., (1988), Pharmaceut. Research 5, 539-549

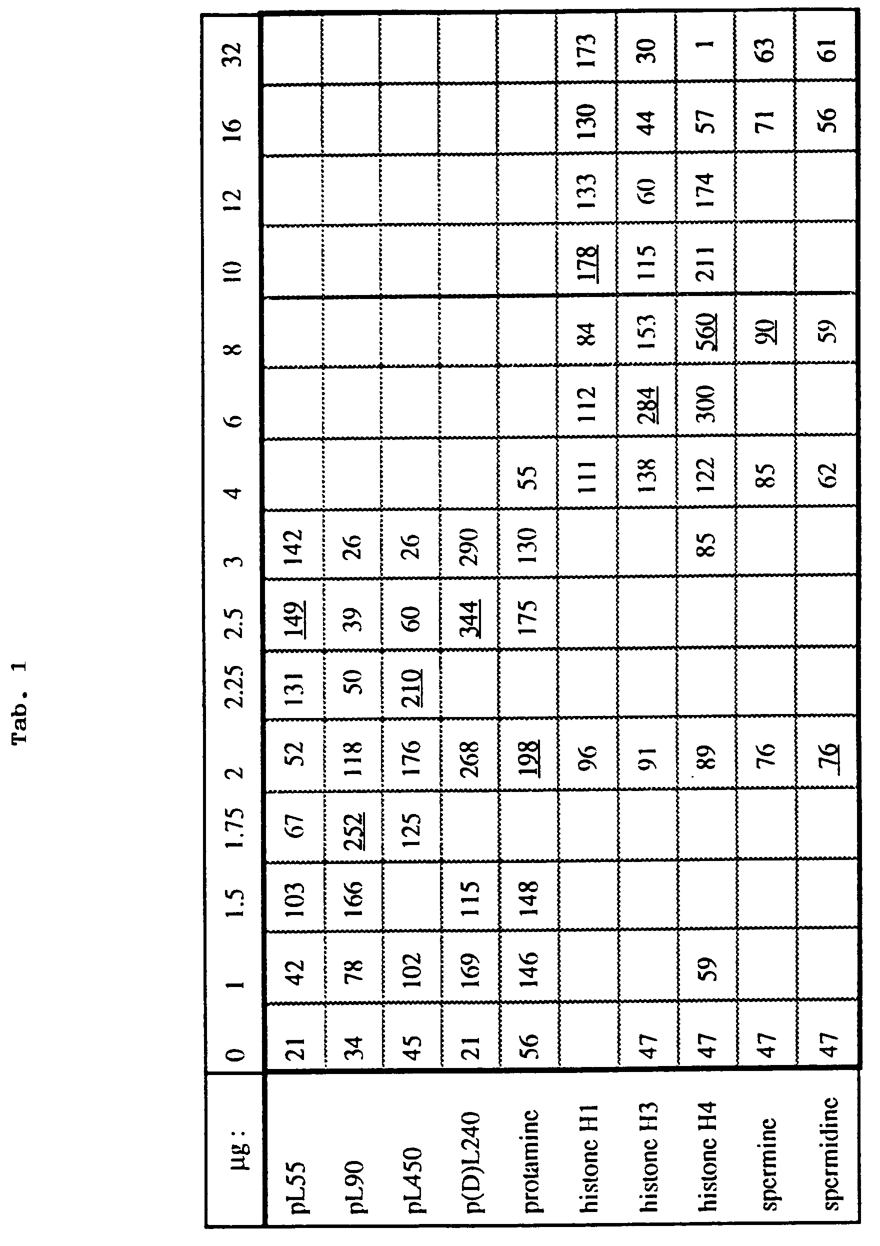

## Patentansprüche

1. Komplexe, die in höhere eukaryotische Zellen aufgenommen werden, enthaltend Nukleinsäure (A), die komplexiert ist mit einem Konjugat aus einem Liganden, der nach Bindung an die Zielzellen über Endozytose oder über Fusion mit der Zellmembran internalisiert wird, und einer an Nukleinsäure bindenden Substanz (B), ausgewählt aus der Gruppe organische Polykationen und interkalierende Substanzen, dadurch gekennzeichnet, daß die Komplexe zusätzlich ein oder mehrere nicht-kovalent gebundene organische Polykationen (C), die gegebenenfalls identisch sind mit dem in dem Konjugat enthaltenen Polykation (B), in einer Menge enthalten, durch welche die durch das Konjugat erzielte Aufnahme in die Zelle und/oder die Expression der Nukleinsäure gesteigert wird.

2. Komplexe nach Anspruch 1, dadurch gekennzeichnet, daß der Ligand über Rezeptor-vermittelte Endozytose in die Zelle aufgenommen wird.

3. Komplexe nach Anspruch 2, dadurch gekennzeichnet, daß der Ligand Transferrin ist.

4. Komplexe nach Anspruch 1, dadurch gekennzeichnet, daß der Ligand an ein Oberflächenantigen der Zielzellen bindet.

5. Komplexe nach Anspruch 4, dadurch gekennzeichnet, daß der Ligand an CD4 bindet.

6. Komplexe nach Anspruch 5, dadurch gekennzeichnet, daß der Ligand ein anti-CD4-Antikörper ist.

7. Komplexe nach Anspruch 5, dadurch gekennzeichnet, daß der Ligand ein virales Protein, vorzugsweise gp120, ist.

8. Komplexe nach Anspruch 1, dadurch gekennzeichnet, daß die Substanz (B) ein homologes organisches Polykation ist.

9. Komplexe nach Anspruch 8, dadurch gekennzeichnet, daß das Polykation Polylysin ist.

10. Komplexe nach Anspruch 1, dadurch gekennzeichnet, daß die Substanz (B) ein Protamin ist.

11. Komplexe nach Anspruch 1, dadurch gekennzeichnet, daß die an Nukleinsäure bindende Substanz (B) ein Ethidiumdimer ist.

12. Komplexe nach einem der Ansprüche 8 bis 11, dadurch gekennzeichnet, daß das nicht-kovalent gebundene Polykation (C) die Eigenschaft hat, die Nukleinsäure zu kondensieren.

13. Komplexe nach Anspruch 12, dadurch gekennzeichnet, daß das Polykation (C) homolog ist.

14. Komplexe nach Anspruch 13, dadurch gekennzeichnet, daß das Polykation (C) Polylysin ist.

15. Komplexe nach Anspruch 1, dadurch gekennzeichnet, daß Substanz (B) und/oder das Polykation (C) mit einer hydrophilen oder lipophilen Gruppierung modifiziert ist.

16. Komplexe nach Anspruch 11 oder 15, dadurch gekennzeichnet, daß das Polykation (C) homolog ist.

17. Komplexe nach Anspruch 16, dadurch gekennzeichnet, daß das Polykation (C) Polylysin ist.

18. Komplexe nach Anspruch 1, dadurch gekennzeichnet, daß das Polykation (C) ein Protamin ist.

19. Komplexe nach Anspruch 1, dadurch gekennzeichnet, daß die im Konjugat enthaltene Substanz (B) Polylysin und das nicht-kovalent gebundene Polykation (C) ein Histon oder eine Mischung von Histonen ist.

20. Komplexe nach Anspruch 19, dadurch gekennzeichnet, daß die Substanz (C) Histon H4 ist.

21. Komplexe nach Anspruch 1, dadurch gekennzeichnet, daß das nicht-kovalent gebundene Polykation (C) das Nicht-Histon-Protein HMGI ist.

22. Komplexe nach einem der Ansprüche 19 bis 21, dadurch gekennzeichnet, daß der Ligand Transferrin ist.

23. Verfahren zur Herstellung von Komplexen nach Anspruch 1, dadurch gekennzeichnet, daß man Lösungen der Komponenten Nukleinsäure, Konjugat und nicht-kovalent gebundenes Polykation mischt.

24. Verfahren zur Herstellung von Komplexen nach einem der Ansprüche 8 bis 14, dadurch gekennzeichnet, daß man das Konjugat mit dem nicht-kovalent gebundenen Polykation mischt und dieses Gemisch mit Nukleinsäure vereinigt.

25. Verfahren zur Herstellung von Komplexen nach einem der Ansprüche 11, 16 und 17, dadurch gekennzeichnet, daß man die Nukleinsäure mit dem Konjugat komplexiert und diesem Komplex das nicht-kovalent gebundene Polykation hinzufügt.

26. Verfahren zum Einbringen von Nukleinsäure in höhere eukaryotische Zellen mittels Endozytose, dadurch gekennzeichnet, daß man aus Nukleinsäure, einem Konjugat aus einem Liganden und einer an Nukleinsäure bindenden Substanz, ausgewählt aus der Gruppe organische Polykationen und interkalierende Substanzen, und einem nicht-kovalent gebundenen organischen Polykation, vorzugsweise unter physiologischen Bedingungen löslichen Komplex nach einem der Ansprüche 1 bis 22 bildet und die Zellen mit diesem Komplex *in vitro* oder *ex vivo* in Berührung bringt.

27. Verfahren nach Anspruch 26, dadurch gekennzeichnet, daß man die Zellen Bedingungen aussetzt, die den Abbau der Nukleinsäure in der Zelle, insbesondere in Lysosomen, inhibieren.

28. Verfahren nach Anspruch 27, dadurch gekennzeichnet, daß man die Zellen mit Chloroquin behandelt.

29. Verfahren nach einem der Ansprüche 26 bis 28, dadurch gekennzeichnet, daß man einen Komplex einsetzt, in dem der Ligand Transferrin ist.

30. Verfahren nach Anspruch 29, dadurch gekennzeichnet, daß man die Zellen Bedingungen aussetzt, unter denen die Transferrin-Rezeptorzahl erhöht wird.

31. Verfahren nach Anspruch 30, dadurch gekennzeichnet, daß man die Zellen vor/und oder gleichzeitig mit der Behandlung mit dem Komplex mit einer oder mehreren Substanzen behandelt, die eine Erhöhung der Transferrin-Rezeptorzahl bewirken, indem sie die Hämkonzentration in der Zelle senken.

32. Verfahren nach Anspruch 31, dadurch gekennzeichnet, daß man die Zellen mit Desferrioxamin behandelt.

33. Pharmazeutische Zubereitung, dadurch gekennzeichnet, daß sie einen oder mehrere der in den Ansprüchen 1 bis 22 definierten Komplexe mit einer therapeutisch wirksamen Nukleinsäure enthält.

34. Pharmazeutische Zubereitung nach Anspruch 33, dadurch gekennzeichnet, daß die Nukleinsäure ein gentherapeutisch wirksames Gen bzw. ein Genabschnitt ist.

35. Pharmazeutische Zubereitung nach Anspruch 34, dadurch gekennzeichnet, daß die Nukleinsäure ein Antisenseoligonukleotid ist oder ein solches enthält.

36. Pharmazeutische Zubereitung nach Anspruch 33, dadurch gekennzeichnet, daß die Nukleinsäure ein Ribozym oder ein dafür kodierendes Gen ist bzw. ein solches enthält.

37. Pharmazeutische Zubereitung nach Anspruch 33, dadurch gekennzeichnet, daß die Nukleinsäure ein Genkonstrukt ist, das einen Abschnitt enthält, von dem Zellfunktionen spezifisch inhibierende RNA-Moleküle transkribierbar sind.

## Claims

1. Complexes which are absorbed into higher eukaryotic cells, containing nucleic acid (A) which is complexed with a conjugate consisting of a ligand which is internalised after binding to the target cells by endocytosis or by fusion with the cell membrane, and a substance (B) which binds to nucleic acid, selected from the group comprising organic polycations and intercalating substances, characterised in that the complexes additionally contain one or more non-covalently bound organic polycations (C) which may possibly be identical to the polycation (B) contained in the conjugate, in a quantity which increases the uptake into the cell achieved by means of the conjugate and/or the expression of the nucleic acid.

2. Complexes according to claim 1, characterised in that the ligand is absorbed into the cell via receptor-mediated endocytosis.

3. Complexes according to claim 2, characterised in that the ligand is transferrin.

4. Complexes according to claim 1, characterised in that the ligand binds to a surface antigen of the target cells.

5. Complexes according to claim 4, characterised in that the ligand binds to CD4.

6. Complexes according to claim 5, characterised in that the ligand is an anti-CD4 antibody.

7. Complexes according to claim 5, characterised in that the ligand is a viral protein, preferably gp120.

8. Complexes according to claim 1, characterised in that substance (B) is a homologous organic polycation.

9. Complexes according to claim 8, characterised in that the polycation is polylysine.

10. Complexes according to claim 1, characterised in that substance (B) is a protamine.

11. Complexes according to claim 1, characterized in that the nucleic acid binding substance (B) is an ethidium dimer.

12. Complexes according to one of claims 8 to 11, characterised in that the non-covalently bound polycation (C) is capable of condensing the nucleic acid.

13. Complexes according to claim 12, characterised in that the polycation (C) is homologous.

14. Complexes according to claim 13, characterised in that the polycation (C) is polylysine.

15. Complexes according to claim 1, characterised in that substance (B) and/or polycation (C) is modified with a hydrophilic or lipophilic grouping.

16. Complexes according to claim 11 or 15, characterised in that the polycation (C) is homologous.

17. Complexes according to claim 16, characterised in that the polycation (C) is polylysine.

18. Complexes according to claim 1, characterised in that the polycation (C) is a protamine.

19. Complexes according to claim 1, characterised in that the substance (B) contained in the conjugate is polylysine and the non-covalently bound polycation (C) is a histone or a mixture of histones.

20. Complexes according to claim 19, characterised in that the substance (C) is histone H4.

21. Complexes according to claim 1, characterised in that the non-covalently bound polycation (C) is the non-histone protein HMGI.

22. Complexes according to one of claims 19 to 21, characterised in that the ligand is transferrin.

23. Process for preparing complexes according to claim 1, characterised in that solutions of the components nucleic acid, conjugate and non-covalently bound polycation are mixed together.

24. Process for preparing complexes according to one of claims 8 to 14, characterised in that the conjugate is mixed with the non-covalently bound polycation and this mixture is combined with nucleic acid.

25. Process for preparing complexes according to one of claims 11, 16 and 17, characterised in that the nucleic acid is complexed with the conjugate and the non-covalently bound polycation is added to this complex.

26. Process for introducing nucleic acid into higher eukaryotic cells by endocytosis, characterised in that a complex which is preferably soluble under physiological conditions according to one of claims 1 to 22 is formed from nucleic acid, a conjugate of a ligand and a substance binding to nucleic acid, selected from the group comprising organic polycations and intercalating substances, and a non-covalently bound organic polycation, and the cells are brought into contact with this complex *in vitro* or *ex vivo*.

27. Process according to claim 26, characterised in that the cells are exposed to conditions which inhibit the breakdown of nucleic acid in the cell, particularly in lysosomes.

28. Process according to claim 27, characterised in that the cells are treated with chloroquin.

29. Process according to one of claims 26 to 28, characterised in that a complex is used in which the ligand is transferrin.

30. Process according to claim 29, characterised in that the cells are exposed to conditions under which the number of transferrin receptors is increased.

31. Process according to claim 30, characterised in that the cells are treated before and/or at the same time as the treatment with the complex with one or more substances which bring about an increase in the number of transferrin receptors by reducing the haem concentration in the cell.

32. Process according to claim 31, characterised in that the cells are treated with desferrioxamine.

33. Pharmaceutical preparation, characterised in that it contains one or more of the complexes defined in claims 1 to 22 with a therapeutically effective nucleic acid.

34. Pharmaceutical preparation according to claim 33, characterised in that the nucleic acid is a gene which is effective in gene therapy or is a gene section.

35. Pharmaceutical preparation according to claim 34, characterised in that the nucleic acid is an antisense oligonucleotide or contains one.

36. Pharmaceutical preparation according to claim 33, characterised in that the nucleic acid is a ribozyme or a gene coding therefor or contains one.

37. Pharmaceutical preparation according to claim 33, characterised in that the nucleic acid is a gene construct containing a section from which RNA molecules which specifically inhibit cell functions can be transcribed.

## Revendications

1. Complexes, qui sont absorbés dans des cellules eucaryotes supérieures, contenant un acide nucléique (A) qui est complexé avec un conjugué constitué par un ligand qui est internalisé après liaison aux cellules cibles par endocytose ou par fusion avec la membrane cellulaire et par une substance (B) qui se lie à l'acide nucléique, choisie dans le groupe des polycations organiques et des substances intercalantes, caractérisés en ce que les complexes contiennent en outre un ou plusieurs polycations organiques (C) liés de manière non covalente, qui sont éventuellement identiques au polycation (B) contenu dans le conjugué, en une quantité par laquelle l'absorption dans la cellule et/ou l'expression de l'acide nucléique obtenue par le conjugué est augmentée.

2. Complexes selon la revendication 1, caractérisés en ce que le ligand est absorbé dans la cellule par endocytose assurée par des récepteurs.

3. Complexes selon la revendication 2, caractérisés en ce que le ligand est la transferrine.

4. Complexes selon la revendication 1, caractérisés en ce que le ligand se lie à un antigène de surface des cellules cibles.

5. Complexes selon la revendication 4, caractérisés en ce que le ligand se lie à CD4.

6. Complexes selon la revendication 5, caractérisés en ce que le ligand est un anticorps anti-CD4.

7. Complexes selon la revendication 5, caractérisés en ce que le ligand est une protéine virale, de préférence gp120.

8. Complexes selon la revendication 1, caractérisés en ce que la substance (B) est un polycation organique homologue.

9. Complexes selon la revendication 8, caractérisés en ce que le polycation est la polylysine.

10. Complexes selon la revendication 1, caractérisés en ce que la substance (B) est une protamine.

11. Complexes selon la revendication 1, caractérisés en ce que la substance (B) qui se lie à l'acide nucléique est un dimère d'éthidium.

12. Complexes selon l'une des revendications 8 à 11, caractérisés en ce que le polycation (C) lié de manière non covalente a la propriété de condenser l'acide nucléique.

13. Complexes selon la revendication 12, caractérisés en ce que le polycation (C) est homologue.

14. Complexes selon la revendication 13, caractérisés en ce que le polycation (C) est la polylysine.

15. Complexes selon la revendication 1, caractérisés en ce que la substance (B) et/ou le polycation (C) est modifié(e) avec un groupement hydrophile ou lipophile.

16. Complexes selon la revendication 11 ou 15, caractérisés en ce que le polycation (C) est homologue.

17. Complexes selon la revendication 16, caractérisés en ce que le polycation (C) est la polylysine.

18. Complexes selon la revendication 1, caractérisés en ce que le polycation (C) est une protamine.

19. Complexes selon la revendication 1, caractérisés en ce que la substance (B) contenue dans le conjugué est la polylysine et le polycation (C) lié de manière non covalente est une histone ou un mélange d'histones.

20. Complexes selon la revendication 19, caractérisés en ce que la substance (C) est l'histone H4.

21. Complexes selon la revendication 1, caractérisés en ce que le polycation (C) lié de manière non covalente est la protéine non-histone HMGI.

22. Complexes selon l'une des revendications 19 à 21, caractérisés en ce que le ligand est la transferrine.

23. Procédé de préparation de complexes selon la revendication 1, caractérisé en ce que l'on mélange des solutions des constituants acide nucléique, conjugué et polycation lié de manière non covalente.

24. Procédé de préparation de complexes selon l'une des revendications 8 à 14, caractérisé en ce que l'on mélange le conjugué avec le polycation lié de manière non covalente et l'on combine ce mélange avec l'acide nucléique.

25. Procédé de préparation de complexes selon l'une des revendications 11, 16 et 17, caractérisé en ce que l'on complexe l'acide nucléique avec le conjugué et on ajoute à ce complexe le polycation lié de manière non covalente.

26. Procédé pour introduire un acide nucléique dans des cellules eucaryotes supérieures par endocytose, caractérisé en ce que l'on forme un complexe selon l'une des revendications 1 à 22 de préférence soluble dans les conditions physiologiques à partir d'acide nucléique, d'un conjugué constitué par un ligand et par une substance qui se lie à l'acide nucléique, choisie dans le groupe des polycations organiques et des substances intercalantes, et d'un polycation organique lié de manière non covalente, et on met les cellules en contact avec ce complexe *in vitro* ou *ex vivo*.

27. Procédé selon la revendication 26, caractérisé en ce que l'on expose les cellules à des conditions qui inhibent la dégradation de l'acide nucléique dans la cellule, en particulier dans les lysosomes.

28. Procédé selon la revendication 27, caractérisé en ce que l'on traite les cellules avec la chloroquine.

29. Procédé selon l'une des revendications 26 à 28, caractérisé en ce que l'on utilise un complexe dans lequel le ligand est la transferrine.

30. Procédé selon la revendication 29, caractérisé en ce que l'on expose les cellules à des conditions dans lesquelles le nombre de récepteurs de la transferrine est augmenté.

31. Procédé selon la revendication 30, caractérisé en ce que, avant et/ou pendant le traitement avec le complexe, on traite les cellules avec une ou plusieurs substances qui provoquent une augmentation du nombre de récepteurs de la transferrine en abaissant la concentration d'hème dans la cellule.

32. Procédé selon la revendication 31, caractérisé en ce que l'on traite les cellules avec la desferrioxamine.

33. Préparation pharmaceutique caractérisée en ce qu'elle contient un ou plusieurs des complexes définis dans les revendications 1 à 22 avec un acide nucléique thérapeutiquement efficace.

34. Préparation pharmaceutique selon la revendication 33, caractérisée en ce que l'acide nucléique est un gène ou un segment de gène efficace du point de vue de la thérapie génique.

35. Préparation pharmaceutique selon la revendication 34, caractérisée en ce que l'acide nucléique est ou contient un oligonucléotide antisens.

36. Préparation pharmaceutique selon la revendication 33, caractérisée en ce que l'acide nucléique est ou contient un ribozyme ou un gène codant celui-ci.

37. Préparation pharmaceutique selon la revendication 33, caractérisée en ce que l'acide nucléique est une construction génique qui contient un segment par lequel des molécules d'ARN inhibant spécifiquement des fonctions cellulaires peuvent être transcrites.
